# EUROPÄISCHE PATENTSCHRIFT

(11) **EP 1 404 637 B1**
(45) Veröffentlichungstag und Bekanntmachung des Hinweises auf die Patenterteilung: **09.09.2009**
(21) Anmeldenummer: 02791437.3
(22) Anmeldetag: 21.06.2002
(51) Int. Cl.: C07C 45/35, C07C 253/26, C07C 5/333

(54) **VERFAHREN ZUR HERSTELLUNG VON PARTIELLEN OXIDATIONSPRODUKTEN UND/ODER PARTIELLEN AMMOXIDATIONSPRODUKTEN WENIGSTENS EINES OLEFINISCHEN KOHLENWASSERSTOFFS**
METHOD FOR PRODUCING PARTIAL OXIDATION PRODUCTS AND/OR PARTIAL AMMOXIDATION PRODUCTS OF AT LEAST ONE OLEFINIC HYDROCARBON
PROCEDE DE FABRICATION DE PRODUITS D'OXYDATION PARTIELLE ET/OU DE PRODUITS D'AMMOXYDATION PARTIELLE D'AU MOINS UN HYDROCARBURE OLEFINIQUE

(30) Priorität: 29.06.2001 DE 10131297
(43) Veröffentlichungstag der Anmeldung: 07.04.2004
(73) Patentinhaber: BASF SE, 67056 Ludwigshafen (DE)
(72) Erfinder: PROLL, Theo, 67098 Bad Dürkheim (DE); MACHHAMMER, Otto, 68163 Mannheim (DE); SCHINDLER, Götz-Peter, 68219 Mannheim (DE); MÜLLER-ENGEL, Klaus, Joachim, 76297 Stutensee (DE)
(86) Internationale Anmeldenummer: PCT/EP2002/006878
(87) Internationale Veröffentlichungsnummer: WO 2003/011804

(56) Entgegenhaltungen:
- EP-A- 0 117 146
- EP-A- 0 328 280
- EP-A- 0 731 080
- WO-A-01/96270
- DE-A- 3 313 573

## Beschreibung

Vorliegende Erfindung betrifft ein Verfahren zur Herstellung von partiellen Oxidationsprodukten und/oder partiellen Ammoxidationsprodukten wenigstens eines olefinischen Kohlenwasserstoffs A', bei dem man
A) in einer ersten Reaktionszone A wenigstens einen paraffinischen Kohlenwasserstoff A einer partiellen Dehydrierung und/oder Oxidehydrierung unter Bildung eines Produktgemisches A, das eine nicht umgesetzte Menge des wenigstens einen Kohlenwasserstoff A und wenigstens einen durch die partielle Dehydrierung und/oder Oxidehydrierung gebildeten olefinischen Kohlenwasserstoff A' enthält, unterwirft,
B) aus dem Produktgemisch A von den darin enthaltenen, von dem wenigstens einen paraffinischen Kohlenwasserstoff A und dem wenigstens eine olefinischen Kohlenwasserstoff A' verschiedenen Bestandteilen gegebenenfalls in einer Abtrennzone eine Teil- oder die Gesamtmenge unter Erhalt eines Produktgemisches A' abtrennt und entweder das Produktgemisch A oder das Produktgemisch A' zur Beschickung einer Oxidations- und/oder Ammoxidationszone B verwendet und in der Oxidations- und/oder Ammoxidationszone B wenigstens den wenigstens einen olefinischen Kohlenwasserstoff A' einer partiellen Oxidation und/oder Ammoxidation unter Bildung eines Produktgemisches B, das als Zielprodukt wenigsten ein partielles Oxidations- und/oder Ammoxidationsprodukt B des wenigsten einen olefinischen Kohlenwasserstoff A' enthält, unterwirft,
C) aus dem Produktgemisch B in einer Aufarbeitungszone C Zielprodukt abtrennt und von dem im Produktgemisch B enthaltenen, nicht umgesetzten wenigstens einen paraffinischen Kohlenwasserstoff A wenigstens eine Teilmenge als paraffinischen Rückführkohlenwasserstoff A in die Reaktionszone A zurückführt und
D) von Zeit zu Zeit in einer Nichtbetriebsphase wenigstens eine Teilmenge der Reaktionszone A nicht zum Zweck der partiellen Dehydrierung und/oder Oxidehydrierung das wenigstens einen paraffinischen Kohlenwasserstoff A betreibt.

Partielle Oxidationsprodukte olefinischer Kohlenwasserstoffe sind wichtige Zwischenprodukte, z.B. zur Herstellung von Polymerisaten.

Unter Oxidehydrierung wird in dieser Schrift eine Dehydrierung verstanden, die durch anwesenden Sauerstoff erzwungen und bei der intermediär kein Wasserstoff gebildet wird.

Unter einem paraffinischen Kohlenwasserstoff soll in dieser Schrift ein gesättigter Kohlenwasserstoff verstanden werden. Unter einem olefinischen Kohlenwasserstoff soll in dieser Schrift ein Kohlenwasserstoff verstanden werden, der wenigstens eine ethylenisch ungesättigte Doppelbindung aufweist (Beispiele sind Propylen, iso-Buten, Butadien).

In der Regel enthalten die paraffinischen und die olefinischen Kohlenwasserstoffe in dieser Schrift nicht mehr als zehn Kohlenstoffatome. Bevorzugt weisen die paraffinischen und die olefinischen Kohlenwasserstoffe in dieser Schrift zwei, drei oder vier Kohlenstoffatome auf. Bevorzugte paraffinische Kohlenwasserstoffe dieser Schrift sind Ethan, Propan und Butane. Bevorzugte olefinische Kohlenwasserstoffe sind in dieser Schrift Propylen (Propen), Ethylen (Ethen) und Butene. Die bevorzugten partiellen Oxidations- und/oder Ammoxidationsprodukte olefinischer Kohlenwasserstoffe sind in dieser Schrift, Acrolein, Acrylsäure, Ethylenoxid, Methacrolein, Methacrylsäure, Propylenoxid, Acrylnitril und Methacrylnitril.

Unter einer vollständigen Oxidation eines olefinischen Kohlenwasserstoffs wird in dieser Schrift verstanden, dass der im olefinischen Kohlenwasserstoff insgesamt enthaltene Kohlenstoff in Oxide des Kohlenstoffs (CO, CO₂) umgewandelt wird. Alle davon verschiedenen Umsetzungen eines olefinischen Kohlenwasserstoffs unter reaktiver Einwirkung von molekularem Sauerstoff werden in dieser Schrift mit dem Begriff Partialoxidation subsummiert. Die zusätzliche reaktive Einwirkung von Ammoniak kennzeichnet die Ammoxidation.

Es ist allgemein bekannt, dass durch partielle Oxidation und/oder Ammoxidation von olefinischen Kohlenwasserstoffen zahlreiche Grundchemikalien erzeugt werden können. Beispielhaft genannt seien die Umsetzung von Propylen zu Acrolein und/oder Acrylsäure (vgl. z.B. DE-A 2 351 151), die Umsetzung von Ethylen zu Ethylenoxid (vgl. z.B. DE-AS 1 254 137, DE-A 2 159 346, EP-A 372 972, WO-89/0710), die Umsetzung von Butadien zu Maleinsäureanhydrid (vgl. z.B. DE-A 2 106 796 und DE-A 1 624 921), die Umsetzung von Butadien zu Vinyloxiran (vgl. z.B. US-A 5 312 931), die Umsetzung von Propylen zu Acrylnitril (vgl. z.B. DE-A 2 351 151), die Umsetzung von iso-Buten zu Methacrolein (vgl. z.B. DE-A 10101695), die Umsetzung von iso-Buten zu Methacrylnitril (vgl. z.B.
US-A 3 882 159) und die Umsetzung von Propylen zu Propylenoxid (vgl. z.B. EP-A 372 972).

In der Regel handelt es sich bei den Herstellverfahren um heterogen katalysierte Gasphasenoxidationen und/oder -ammoxidationen. Die Ammoxidation unterscheidet sich dabei von der Oxidation üblicherweise durch ein Beisein von Ammoniak. Bei geschickter Wahl des Ammoniakgehaltes können die Gasphasenoxidation und die Ammoxidation eines olefinischen Kohlenwasserstoffs auch gleichzeitig in einem Gasgemisch durchgeführt werden. Als Oxidationsmittel wird normalerweise molekularer Sauerstoff verwendet, der zum Beispiel in reiner Form oder im Gemisch mit sich bezüglich der Partialoxidation im wesentlichen inert verhaltenden Gasen (z.B. Luft) dem Reaktionsgasausgangsgemisch zugesetzt werden kann. Als Katalysatoren werden in der Regel Multimetalloxide eingesetzt und häufig sind im Reaktionsgasausgangsgemisch die Reaktanden molekularer Sauerstoff und olefinischer Kohlenwasserstoff durch ein Inertgas (z.B. N₂, H₂O, CO, CO₂, He und/oder Ar etc.) verdünnt.

Es sind aber auch Verfahren der Flüssigphasenpartialoxidation und/oder -ammoxidation von olefinischen Kohlenwasserstoffen bekannt.

Als olefinischer Ausgangskohlenwasserstoff wird für die im Stand der Technik gewürdigten Partialoxidationen und/oder -ammoxidationen üblicherweise ein sogenannter roher olefinischer Kohlenwasserstoff verwendet, der normalerweise neben Nebenkomponenten zu wenigstens 90 % seines Gewichtes an olefinischem Kohlenwasserstoff enthält. Häufig beträgt der Gehalt an olefinischem Kohlenwasserstoff sogar wenigstens 95 % seines Gewichtes oder wenigstens 99 % seines Gewichtes.

Die Gewinnung solcher vergleichsweise reiner roher olefinischer Kohlenwasserstoffe ist relativ aufwendig und kostspielig. Sie geht meist von paraffinischen Kohlenwasserstoffen aus und beinhaltet normalerweise wenigstens eine Reinigungsstufe in welcher nicht umgesetzter paraffinischer Kohlenwasserstoff von gebildetem olefinischem Kohlenwasserstoff mittels physikalischer Verfahren abgetrennt wird (vgl. z.B. DE-A 3521458). Diese Abtrennung ist in aller Regel investitionsintensiv und in Folge der Ähnlichkeit von olefinischen und paraffinischen Kohlenwasserstoffen sehr energieintensiv. Sie wird daher üblicherweise nur im Verbund mit Raffineriecrackern und Steamcrackern angewendet und rechnet sich nur deshalb, weil die überwiegende Menge der so gewonnenen rohen olefinischen Kohlenwasserstoffe für nachfolgende Polymerisationen einerseits in großen Mengen benötigt wird und dabei andererseits eine hohe Wertschöpfung erfährt.

Der von diesen rohen olefinischen Kohlenwasserstoffen in Partialoxidationen und/oder -ammoxidationen fließende Anteil ist von eher untergeordneter Bedeutung und läuft als eher untergeordneter Nebenbedarfsstrom quasi neben her. Dies hat zur Konsequenz, daß solchermaßen erzeugter roher olefinischer Kohlenwasserstoff auch für Partialoxidationen und/oder -ammoxidationen noch einen akzeptablen Rohstoffpreis aufweist.

Dieser Rohstoffpreis ließe sich nun aber signifikant dadurch senken, daß auf die beschriebene Trennung von paraffinischem Kohlenwasserstoff und gebildetem olefinischem Kohlenwasserstoff verzichtet und der dabei anfallende rohe olefinische Kohlenwasserstoff als solcher für die nachfolgende Partialoxidation und/oder -ammoxidation des olefinischen Kohlenwasserstoffs verwendet wird. Dies ist prinzipiell möglich, weil sich paraffinische Kohlenwasserstoffe im Bezug auf, insbesondere in der Gasphase heterogen katalysierte, Partialoxidationen und/oder -ammoxidationen von olefinischen Kohlenwasserstoffen im Regelfall im wesentlichen inert verhalten. D.h., sie wirken überwiegend wie ein inerter Verdünner, der den Oxidations- und/oder Ammoxidationsprozeß im wesentlichen unverändert durchläuft. Eine solche Verfahrensweise kann aber nur dann wirtschaftlich sein, wenn der die Partialoxidation und/oder -ammoxidation des olefinischen Kohlenwasserstoff verlassende paraffinische Kohlenwasserstoff vom Zielprodukt abgetrennt und anschließend einer anderen Weiterverwendung zugeführt wird, wie es z.B. die EP-A 731 080 vorschlägt. Eine Rückführung in den Cracker wäre dagegen nur wenig sinnvoll, würden die den paraffinischen Kohlenwasserstoff begleitenden Nebenkomponenten den Crackerbetrieb im Normalfall doch empfindlich stören.

Als Problemlösung wurde deshalb auch schon vorgeschlagen, den für die partielle Oxidation und/oder Ammoxidation benötigten olefinischen Kohlenwasserstoff durch eine partielle Dehydrierung und/oder Oxidehydrierung eines paraffinischen Kohlenwasserstoffs zu erzeugen, aus dem Produktgemisch gegebenenfalls eine Teil- oder die Gesamtmenge der vom enthaltenen olefinsichen Kohlenwasserstoff und dem noch verbliebenen paraffinischen Kohlenwasserstoff verschiedenen Bestandteile abzutrennen und es dann, den olefinischen Kohlenwasserstoff in Begleitung des verbliebenen paraffinischen Kohlenwasserstoff aufweisend, zur Beschickung einer partiellen Oxidation und/oder Ammoxidation des olefinischen Kohlenwasserstoff zu verwenden. Aus dem Produktgemisch der partiellen Oxidation und/oder Ammoxidation wird dann Zielprodukt abgetrennt und danach der in diesem Produktgemisch enthaltene paraffinische Kohlenwasserstoff als paraffinischer Rückführkohlenwasserstoff wenigstens teilweise in die Dehydrierung und/oder Oxidehydrierung rückgeführt.

Nachteilig an einer solchen Verfahrensweise ist jedoch, daß eine Dehydrierung und/oder Oxidehydrierung paraffinischer Kohlenwasserstoffe im Regelfall immer mit einer Abscheidung von Kohlenstoffablagerungen einhergeht, die von Zeit zu Zeit entfernt werden müssen. Um in solchen Nichtbetriebsphasen zu verhindern, daß auch die partielle Oxidation und/oder Ammoxidation unterbrochen werden muß, wird die Dehydrierung und/oder Oxidehydrierung üblicherweise mit wenigstens zwei Dehydrier- und/oder Oxidehydrierreaktoren (die gemeinsam eine Dehydrier- und/oder Oxidehydrierzone bilden) durchgeführt, deren Nichtbetriebsphasen zeitlich phasenverschoben durchgeführt werden.

Nachteilig an einer solchen Gestaltungsvariante ist jedoch, daß sie des Investments in wenigstens zwei Dehydrier- und/oder Oxidehydrierreaktoren bedarf, wodurch die Gesamtwirtschaftlichkeit in Frage gestellt wird.

An vielen Standorten werden nun aber bereits partielle Oxidationen und/oder Ammoxidationen von olefinischen Kohlenwasserstoffen betrieben, wobei der olefinische Kohlenwasserstoff aus einer anderen, im Regelfall noch weitere Kunden beliefernde, Quelle stammt.

In der PCT/EP/01/06528 wird insbesondere für eine solche Konstellation vorgeschlagen, als Quelle des benötigten olefinischen Kohlenwasserstoffs für den Normalbetrieb der partiellen Oxidation und/oder Ammoxidation des olefinischen Kohlenwasserstoffs eine Dehydrier- und/oder Oxidehydrierzone wie oben stehend beschrieben zu implementieren und während der Nichtbetriebsphase wenigstens eines Teils dieser Zone den Produktionsausfall an olefinischem Kohlenwasserstoff aus der herkömmlichen Quelle zu decken. Durch eine solche Verfahrensweise ließe sich der Reaktorbedarf für die Dehydrier- und/oder Oxidehydrierzone minimieren. Nachteilig an einer solchen Verfahrensweise ist jedoch, daß ein aus den herkömmlichen Quellen stammender olefinischer Kohlenwasserstoff im Regelfall einen wesentlich kleineren Anteil an paraffinischem Kohlenwasserstoff aufweist als ein aus der obenstehend beschriebenen Dehydrier- und/oder Oxidehydrierzone stammender olefinischer Kohlenwasserstoff.

Als Konsequenz daraus würde die Anlage zur partiellen Oxidation und/oder Ammoxidation (einschließlich der Anlage zur Aufarbeitung ihres Produktgemisches) während der Nichtbetriebsphase wenigstens eines Teils der Dehydrier- und/oder Oxidehydrierzone ihren normalen stationären Betriebspunkt stets in weitgehendem Umfang verlassen müssen, was einen Regelmehraufwand bedingt.

Aufgabe der vorliegenden Erfindung war es daher, eine Verfahrensweise zur Verfügung zu stellen, die den genannten Nachteil nicht mehr oder nur noch in einem geringeren Umfang aufweist. Erfindungsgemäß wurde nun gefunden, daß dies zum Beispiel dadurch möglich ist, daß ab Beginn der Nichtbetriebsphase wenigstens eines Teils der Dehydrier- und/oder Oxidehydrierzone der für diesen Teil vorgesehene paraffinische Rückführkohlenwasserstoff wenigstens teilweise nicht über die Dehydrier- und/oder Oxidehydrierzone in die Oxidations- und/oder Ammoxidationszone rückgeführt und nachfolgend innerhalb der Nichtbetriebsphase so immer wieder in diesem kleinen Rückführkreis gefahren wird. Selbstverständlich könnte man ab Beginn der Nichtbetriebsphase auch einen Teil des für den nicht betriebenen Teil der Dehydrier- und/oder Oxidehydrierzone vorgesehenen-paraffinischen Rückführkohlenwasserstoff zunächst durch einen Auslaß abführen und der Oxidations- und/oder Ammoxidationszone dafür eine entsprechende Frischmenge an paraffinischem Kohlenwasserstoff zuführen, die nachfolgend wenigstens teilweise im kleinen Kreis geführt wird.

Als erfindungsgemäße Lösung der gestellten Aufgabe wird somit ein Verfahren zur Herstellung von partiellen Oxidationsprodukten und/oder partiellen Ammoxidationsprodukten wenigstens eines olefinischen Kohlenwasserstoffs A', bei dem man
A) in einer ersten Reaktionszone A wenigstens einen paraffinischen Kohlenwasserstoff A einer partiellen Dehydrierung und/oder Oxidehydrierung unter Bildung eines Produktgemisches A, das eine nicht umgesetzte Menge des wenigstens einen paraffinischen Kohlenwasserstoff A und wenigstens einen durch die partielle Dehydrierung und/oder Oxidehydrierung gebildeten olefinischen Kohlenwasserstoff A' enthält, unterwirft,
B) aus dem Produktgemisch A von den darin enthaltenen, von dem wenigstens einen paraffinischen Kohlenwasserstoff A und dem wenigstens einen olefinischen Kohlenwasserstoff A' verschiedenen Bestandteilen gegebenenfalls eine in einer (ersten) Abtrennzone (Abtrennvorrichtung) eine Teil- oder die Gesamtmenge unter Erhalt eines Produktgemisches A' abtrennt und entweder das Produktgemisch A oder das Produktgemisch A' zur Beschickung einer Oxidations- und/oder Ammoxidationszone B verwendet und in der Oxidations- und/oder Ammoxidationszone B wenigstens den wenigstens einen olefinischen Kohlenwasserstoff A' einer partiellen Oxidation- und/oder Ammoxidation unter Bildung eines Produktgemisches B, das als Zielprodukt wenigstens ein partielles Oxidations- und/oder Ammoxidationsprodukt B des wenigstens einen olefinischen Kohlenwasserstoff A' enthält, unterwirft,
C) aus dem Produktgemisch B in einer Aufarbeitungszone C Zielprodukt abtrennt und von dem im Produktgemisch B enthaltenen, nicht umgesetzten wenigstens einen paraffinischen Kohlenwasserstoff A wenigstens eine Teilmenge als Rückführkohlenwassrestoff A (als Bestandteil eines Rückführstromes) in die Reaktionszone A zurückführt und
D) von Zeit zu Zeit in einer Nichtbetriebsphase wenigstens eine Teilmenge der Reaktionszone A nicht zum Zweck der partiellen Dehydrierung und/oder Oxidehydrierung des wenigstens einen paraffinischen Kohlenwasserstoff A betreibt,
zur Verfügung gestellt, das dadurch gekennzeichnet ist, dass man während der Nichtbetriebsphase der wenigstens einen Teilmenge der Reaktionszone A die Oxidations- und/oder Ammoxidationszone B weiter betreibt und dabei den mit der Nichtbetriebsphase verbundenen Produktionsausfall an dem wenigstens einen olefinischen Kohlenwasserstoff A' dadurch wenigstens teilweise kompensiert, dass man der Oxidations- und/oder Ammoxidationszone B wenigstens einen aus einer anderen Quelle als der Reaktionszone A stammenden olefinischen Kohlenwasserstoff A' und gegebenenfalls einen oder mehrere aus einer anderen Quelle als der Reaktionszone A (bevorzugt ist eine Herstellung ohne eine solche Neuzuführung an paraffinischem Kohlenwasserstoff A) stammenden paraffinischen Kohlenwasserstoff A zuführt und von dem dann im Produktgemisch B der Oxidations- und/oder Ammoxidationszone B befindlichen wenigstens einen paraffinischen Kohlenwasserstoff A wenigstens eine Teilmenge als paraffinischen Rückführkohlenwasserstoff A (als Bestandteil des Rückführstromes) nicht über die Reaktionszone A (d.h., unter Ausschluss der Reaktionszone A) in die Oxidations- und/oder Ammoxidationszone B rückführt. D.h., die letztgenannte Rückführung wird erfindungsgemäß so durchgeführt, daß auf dem Rückführweg keine Dehydrierung und/oder Oxidehydrierung durchlaufen wird.

Wird zwischen der Reaktionszone A und der Reaktionszone B aus dem Produktgemisch A von den darin enthaltenen, von dem wenigstens einen paraffinischen Kohlenwasserstoff A und dem wenigstens einen olefinischen Kohlenwasserstoff A' verschiedenen Bestandteilen in einer Abtrennvorrichtung (Abtrennzone) eine Teil- oder die Gesamtmenge abgetrennt, erfolgt beim erfindungsgemäßen Verfahren die Zufuhr das aus einer anderen Quelle als der Reaktionszone A stammenden wenigstens einen olefinischen Kohlenwasserstoff A' bevorzugt so, daß dieser die Abtrennvorrichtung (Abtrennzone) durchläuft.

Außerdem ist es in diesem Fall bevorzugt, die Teilmenge des paraffinischen Rückführkohlenwasserstoff A, die nicht über die Reaktionszone A in die Reaktionszone B (als Bestandteil des Rückführstromes) rückgeführt wird, so in die Reaktionszone B rückzuführen, daß sie die vorgenannte Abtrennvorrichtung (Abtrennzone) durchläuft.

Erfindungsgemäß kann die Reaktionszone A zwei, drei, vier oder mehr Einzelreaktoren für die partielle Dehydrierung und/oder Oxidehydrierung umfassen.

Erfindungsgemäß bevorzugt umfaßt die Reaktionszone A nur einen Reaktor für die partielle Dehydrierung und/oder Oxidehydrierung.

Ferner wird das erfindungsgemäße Verfahren bevorzugt so durchgeführt, daß ab Beginn der Nichtbetriebsphase der wenigstes einen Teilmenge der Reaktionszone A der für diese Teilmenge vorgesehene Anteil des wenigstens einen paraffinischen Rückführkohlenwasserstoff A wenigstens zur Hälfte, besser zu wenigstens 75 % und ganz besonders bevorzugt vollständig der Oxidations- und/oder Ammoxidationszone B nicht mehr über die Reaktionszone A rückgeführt wird. Da dieser Anteil des wenigstens einen paraffinischen Rückführkohlenwasserstoff A sich bezüglich der partiellen Oxidation und/oder Ammoxidation des wenigstens einen olefinischen Kohlenwasserstoff A' im wesentlichen inert verhält, bleibt er während der partiellen Oxidation und/oder Ammoxidation im wesentlichen unverändert erhalten und kann daher so lange in diesem kleinen Kreis (der große Kreis führt über die Reaktionszone A) gefahren werden, bis die Nichtbetriebsphase der wenigstens einen Teilmenge der Reaktionszone A beendet ist. Auf diese Weise ist es möglich, den Betriebszustand der partiellen Oxidation und/oder Ammoxidation einschließlich der erforderlichen Aufarbeitung zur Abtrennung des wenigstens einen Zielproduktes während der Nichtbetriebsphase der wenigstens einen Teilmenge der Reaktionszone A weitgehend oder vollständig unverändert aufrechtzuerhalten.

Erfindungsgemäß wird man also aus dem Produktgemisch B in einer Aufarbeitungszone C unter Erhalt eines Restproduktes B Zielprodukt abtrennen (in der Regel wird man die Gesamtmenge an Zielprodukt abtrennen). Aus dem Restproduktgemisch B kann man dann gegebenenfalls in einer (weiteren, einer zweiten) Abtrennzone (Abtrennstufe) im Restprodukt B enthaltene, vom wenigstens einen paraffinischen Kohlenwasserstoff A verschiedene, Nebenkomponenten unter Erhalt eines den wenigstens einen paraffinischen Kohlenwasserstoff A enthaltenden Restproduktes B teilweise oder vollständig abtrennen und so im Normalbetrieb (d.h., in Abwesenheit einer Nichtbetriebsphase) entweder das Restproduktgemisch B als solches oder das Restproduktgemisch B' als wenigstens einen paraffinischen Rückführkohlenwasserstoff A enthaltenden Rückführstrom in die Reaktionszone A zurückführen (erfindungsgemäß bevorzugt wird nur die erste oder die zweite Abtrennzone angewandt; bevorzugt wird nur die erste Abtrennzone angewendet). Während der Nichtbetriebsphase wird man demgegenüber dann erfindungsgemäß entweder das Restproduktgemisch B oder das Restproduktgemisch B' als wenigstens einen paraffinischen Rückführkohlenwasserstoff A enthaltenden Rückführstrom wenigstens teilweise (bevorzugt vollständig) unter Ausschluß (unter Umgehung) der Reaktionszone A in die Oxidations- und/oder Ammoxidationszone B rückführen. Vorzugsweise führt diese Rückführung über die erste Abtrennzone, falls eine solche angewandt wird.

D.h., umfaßt die Reaktionszone A für die Durchführung des erfindungsgemäßen Verfahrens lediglich einen Reaktor A für die partielle Dehydrierung und/oder Oxidehydrierung , wird man das erfindungsgemäße Verfahren in zweckmäßiger Weise wie folgt durchführen:

Im Reaktor A wird man wenigstens einen paraffinischen Kohlenwasserstoff A einer partiellen Dehydrierung und/oder Oxidehydrierung unterwerfen, wobei ein Produktgemisch A erhalten wird, das eine nicht umgesetzte Menge des wenigstens einen paraffinischen Kohlenwasserstoff A und wenigstens einen durch die partielle Dehydrierung und/oder Oxidehydrierung gebildeten olefinischen Kohlenwasserstoff A' enthält.

Aus dem Produktgemisch A wird man dann von den darin enthaltenen, von dem wenigstens einen paraffinischen Kohlenwasserstoff A und dem wenigstens einen olefinischen Kohlenwasserstoff A' verschiedenen Bestandteilen gegebenenfalls in einer (ersten) Abtrennvorrichtung (Abtrennzone) eine Teil- oder die Gesamtmenge unter Erhalt eines Produktgemisches A' abtrennen und dann entweder das Produktgemisch A oder das Produktgemisch A' zur Beschickung einer Oxidations- und/oder Ammoxidationszone B verwenden.

Im Regelfall (und dies gilt ganz allgemein für die erfindungsgemäße Verfahrensweise) wird das Beschickungsgemisch A" der Oxidations- und/oder Ammoxidationszone B nicht nur das Produktgemisch A bzw. A' umfassen. Vielmehr wird man dem Produktgemisch A bzw. A' zur Erzeugung des Beschickungsgemisches A" im Normalfall zusätzliche Bestandteile wie z.B. Oxidationsmittel, Ammoniak, inerte Verdünnungsmittel etc. zuführen. Selbstverständlich können dem Beschickungsgemisch A" zusätzliche olefinische und/oder paraffinische Kohlenwasserstoffe A' bzw. A aus von der Reaktionszone A verschiedenen Quellen zugeführt werden, um eine besonders bevorzugte Zusammensetzung des Beschickungsgemisches A" zu erzeugen.

In der Oxidations- und/oder Ammoxidationszone B (Reaktionszone B) wird dann der im Beschickungsgemisch A" befindliche wenigstens eine olefinische Kohlenwasserstoff A' unter Erhalt eines Produktgemisches B partiell oxidiert und/oder ammoxidiert. Das Produktgemisch B enthält als wenigstens ein Zielprodukt wenigstens ein partielles Oxidations- und/oder Ammoxidationsprodukt B des wenigstens einen olefinischen Kohlenwasserstoff A' und von dem nicht umgesetzten wenigstens einen paraffinischen Kohlenwasserstoff A.

In einer Aufarbeitungszone C wird man dann aus dem Produktgemisch B Zielprodukt abtrennen und anschließend wenigstens eine Teilmenge (bevorzugt wenigstens die Hälfte, besser wenigstens 75 % und ganz besonders bevorzugt die Gesamtmenge) des nicht umgesetzten, im Produktgemisch B enthaltenen, wenigstens einen paraffinischen Kohlenwasserstoff A (Rückführkohlenwasserstoff A) als Bestandteil eines Rückführstromes R in den Reaktor A zurückführen.

Selbstverständlich kann der Rückführstrom R neben dem wenigstens einen paraffinischen Rückführkohlenwasserstoff A noch andere Bestandteile enthalten. Als diese anderen Bestandteile (Nebenkomponenten) kommen beispielsweise Nebenprodukte aus der partiellen Oxidehydrierung und/oder Dehydrierung, Nebenprodukte aus der partiellen Oxidation und/oder Ammoxidation, nicht vollständig verbrauchtes Oxidationsmittel, gegebenenfalls nicht vollständig abgetrenntes Zielprodukt sowie eventuell nicht umgesetzte Anteile des wenigstens einen olefinischen Kohlenwasserstoff A' sowie Verunreinigungen der eingesetzten Rohstoffe in Betracht.

Selbstverständlich können diese Nebenkomponenten bei Bedarf aus dem Rückführstrom R vor dessen Rückführung in den Reaktor A auch teilweise oder im wesentlichen vollständig abgetrennt (in einer zweiten Abtrennzone) werden. Dann wird man den Rückführstrom R durch frischen wenigstens einen paraffinischen Kohlenwasserstoff A und gegebenenfalls weitere für die Dehydrierung und/oder Oxidehydrierung benötigte Bestandteile ergänzen und den Produktionskreis von neuem durchlaufen.

Wenn nun ab irgendeinem Zeitpunkt der Reaktor A für eine gewisse Zeitspanne nicht mehr zum Zweck der Dehydrierung betrieben wird, würde man ab diesem Zeitpunkt und für diese Zeitspanne erfindungsgemäß den paraffinischen Rückführkohlenwasserstoff A als Bestandteil des Rückführstromes R nicht mehr in die Reaktionszone A sondern wenigstens teilweise, bevorzugt wenigstens zur Hälfte, besser zu wenigstens 75 % und besonders bevorzugt vollständig auf einem nicht über die Reaktionszone A (auf einem nicht über eine Dehydrierung und/oder Oxidehydrierung) führenden Weg in die Oxidations- und/oder Ammoxidationszone B (als Bestandteil dessen Beschickungsgemisches A") rückführen. Beispielsweise kann die Rückführung des paraffinischen Rückführkohlenwasserstoff A (als Bestandteil des Rückführstromes R) dann im wesentlichen unmittelbar in die Oxidations- und/oder Ammoxidationszone B (Reaktionszone B) hinein (in das Beschickungsgemisch A" hinein) erfolgen.

Wird zu Zeiten, in denen der Reaktor A zum Zweck der Dehydrierung und/oder Oxidehydrierung betrieben wird, aus dem Produktgemisch A in einer (ersten) Abtrennvorrichtung (Abtrennzone) wenigstens ein Teil der von dem wenigstens einen olefinischen Kohlenwasserstoff A' und dem verbliebenen wenigstens einen paraffinischen Kohlenwasserstoff A verschiedenen Bestandteile abgetrennt, bevor es als Produktgemisch A' zur Beschickung der Reaktionszone B verwendet wird, wird in der Zeitspanne, in der der Reaktor A nicht zum Zweck der Dehydrierung und/oder Oxidehydrierung betrieben wird, die Teilmenge des Rückführkohlenwasserstoff A (des Rückführstromes R), die nicht über den Reaktor A in die Reaktionszone B rückgeführt wird, vorteilhaft so in die Reaktionszone B rückgeführt, daß sie die (erste) Abtrennvorrichtung (Abtrennzone) durchläuft.

Den für das Beschickungsgasgemisch A" benötigten wenigstens einen olefinischen Kohlenwasserstoff A' würde man in der genannten Zeitspanne wenigstens teilweise, bevorzugt wenigstens zur Hälfte, besser zu wenigstens 75 % und besonders bevorzugt vollständig aus einer anderen Quelle als dem Reaktor A beziehen und der Reaktionszone B zuführen.

Im vorgenannten Fall ist es dabei erfindungsgemäß bevorzugt, die Zufuhr des aus einer anderen Quelle als dem Reaktor A stammenden wenigstens einen olefinischen Kohlenwasserstoff A' so vorzunehmen, daß dieser ebenfalls die (erste) Abtrennvorrichtung durchläuft.

Handelt es sich bei dem wenigsten einen olefinischen Kohlenwasserstoff A' um Propylen und bei dem wenigstens einen paraffinischen Kohlenwasserstoff um Propan, steht als eine andere solche Quelle beispielsweise käuflich erwerbliches Cracker-Propylen der nachfolgenden Reinheitsgrade zur Verfügung:
a) Polymer grade Propylen:

| | |
|---|---|
| ≥ | 99,6 Gew.-% Propen, |
| ≤ | 0,4 Gew.-% Propan, |
| ≤ | 300 Gew.ppm Ethan und/oder Methan, |
| ≤ | 5 Gew.ppm C₄-Kohlenwasserstoffe, |
| ≤ | 1 Gew.ppm Acetylen, |
| ≤ | 7 Gew.ppm Ethylen, |
| ≤ | 5 Gew.ppm Wasser, |
| ≤ | 2 Gew.ppm O₂, |
| ≤ | 2 Gew.ppm Schwefel enthaltende Verbindungen (berechnet als Schwefel), |
| ≤ | 1 Gew.ppm Chlor enthaltende Verbindungen (berechnet als Chlor), |
| ≤ | 5 Gew.ppm CO₂, |
| ≤ | 5 Gew.ppm CO, |
| ≤ | 10 Gew.ppm Cyclopropan, |
| ≤ | 5 Gew.ppm Propadien und/oder Propin, |
| ≤ | 10 Gew.ppm C_{≥5}-Kohlenwasserstoff und |
| ≤ | 10 Gew.ppm Carbonylgruppen enthaltende Verbindungen (berechnet als Ni(CO)₄); |

b) Chemical grade Propylen:

| | |
|---|---|
| ≥ | 94 Gew.-% Propen, |
| ≤ | 6 Gew.-% Propan, |
| ≤ | 0,2 Gew.-% Methan und/oder Ethan, |
| ≤ | 5 Gew.ppm Ethylen, |
| ≤ | 1 Gew.ppm Acetylen, |
| ≤ | 20 Gew.ppm Propadien und/oder Propin, |
| ≤ | 100 Gew.ppm Cyclopropan, |
| ≤ | 50 Gew.ppm Buten, |
| ≤ | 50 Gew.ppm Butadien, |
| ≤ | 200 Gew.ppm C₄-Kohlenwasserstoffe, |
| ≤ | 10 Gew.ppm C_{≥5}-Kohlenwasserstoffe, |
| ≤ | 2 Gew.ppm Schwefel enthaltende Verbindungen (berechnet als Schwefel), |
| ≤ | 0,1 Gew.ppm Sulfide (berechnet als H₂S), |
| ≤ | 1 Gew.ppm Chlor enthaltende Verbindungen (berechnet als Chlor), |
| ≤ | 1 Gew.ppm Chloride (berechnet als Cl^{Θ} ) und |
| ≤ | 30 Gew.ppm Wasser. |

Auf die beschriebene Art und Weise kann so auch während einer Unterbrechung der Dehydrierung und/oder Oxidehydrierung im Reaktor A ohne allzu großen investiven Aufwand die Zusammensetzung des Beschickungsgemisches A" und damit der Betriebszustand der Oxidations- und/oder Ammonoxidationszone B sowie der Aufarbeitungszone C weitestgehend unverändert gehalten werden.

Bevorzugt handelt es sich beim erfindungsgemäßen Verfahren bei dem wenigstens einen paraffinischen Kohlenwasserstoff A um Ethan, Propan, n-Butan und/oder iso-Butan. An seine Reinheit werden keine besonderen Anforderungen gestellt.

Die partielle Dehydrierung und/oder Oxidehydrierung des wenigstens einen paraffinischen Kohlenwasserstoff A kann in dem Fachmann an sich bekannter Weise durchgeführt werden, wie es für den Fall des Propans beispielsweise in der DE-A 19 837 517, der DE-A 19 837 519, der DE-A 19 837 520, der EP-A 117 146, der DE-A 3 313 573, der US-A 3,161,670, der DE-A 10028582, der PCT/EP/01/06708, der EP-A 328 280, der EP-A 193 310, der EP-A 372 972 und in der US-A 4,849,538 beschrieben ist. Sie kann als homogene und/oder heterogen katalysierte Oxidehydrierung oder als heterogen katalysierte Dehydrierung ausgeführt sein. Üblicherweise wird sie bei erhöhten Temperaturen in der Gasphase durchgeführt. In entsprechender Weise kann die partielle Oxidation und/oder Ammoxidation des wenigstens einen paraffinischen Kohlenwasserstoff A in dem Fachmann an sich bekannter Weise durchgeführt werden.

Üblicherweise wird sie ebenfalls in der Gasphase als heterogen katalysierte partielle Oxidation und/oder Ammoxidation durchgeführt, wobei als Katalysatoren feste Multimetalloxide verwendet werden. Konkrete Ausgestaltungen finden sich ebenfalls im vorstehend bezüglich der Ausführung der partiellen Dehydrierung und/oder Oxidehydrierung herangezogenen Stand der Technik.

Als Zielprodukte sind für die erfindungsgemäße Verfahrensweise vor allem Acrolein, Acrylsäure, Methacrolein, Methacrylsäure, Maleinsäureanhydrid, Acrylnitril, Methacrylnitril, Vinyloxiran, Propylenoxid und Ethylenoxid von Interesse.

Ihre Abtrennung aus dem Produktgemisch B kann in der Aufarbeitungszone C ebenfalls in dem Fachmann an sich bekannter Weise erfolgen. Nähere Ausführungen diesbezüglich finden sich ebenfalls im vorstehend bezüglich der Ausführung der partiellen Dehydrierung und/oder Oxidehydrierung herangezogenen Stand der Technik.

Zweckmäßigerweise wird beim erfindungsgemäßen Verfahren in der Reaktionszone A im wesentlichen jeweils nur ein paraffinischer Kohlenwasserstoff A dehydriert und/oder oxidehydriert. Dies ist insbesondere dann möglich, wenn dafür ein roher paraffinischer Kohlenwasserstoff A eingesetzt wird, der im wesentlichen keine anderen paraffinischen Kohlenwasserstoffe enthält.

Einige besondere Ausgestaltungsmöglichkeiten des erfindungsgemäßen Verfahrens seien nachfolgend am Beispiel einer erfindungsgemäßen Herstellung von Acrolein und/oder Acrylsäure ausgehend von Propan erläutert. Sie sind in analoger Weise auf die anderen in dieser Schrift genannten erfindungsgemäßen Verfahren übertragbar und anwendbar.

Im Fall einer Oxidehydrierung des Propan kann diese als homogene und/oder heterogen katalysierte Oxidehydrierung von Propan zu Propen mit molekularem Sauerstoff in der Gasphase durchgeführt werden. Als Quelle des molekularen Sauerstoff kann dabei Luft, reiner molekularer Sauerstoff oder an molekularem Sauerstoff angereicherte Luft eingesetzt werden.

Gestaltet man die Reaktionszone A als eine homogene Oxidehydrierung, so läßt sich diese prinzipiell so durchführen, wie es z.B. in den Schriften US-A 3,798,283, CN-A 1 105 352, Applied Catalysis, 70(2)1991, S. 175-187, Catalysis Today 13, 1992, S. 673-678 und in der Anmeldung DE-A 19 622 331 beschrieben ist. Eine zweckmäßige Sauerstoffquelle ist Luft. Die Temperatur der homogenen Oxidehydrierung wird zweckmäßigerweise im Bereich von 300 bis 700°C, vorzugsweise im Bereich von 400 bis 600°C, besonders bevorzugt im Bereich von 400 bis 500°C liegend gewählt. Der Arbeitsdruck kann 0,5 bis 100 bar, insbesondere 1 bis 10 bar betragen. Die Verweilzeit liegt üblicherweise bei 0,1 beziehungsweise 0,5 bis 20 Sekunden, vorzugsweise bei 0,1 beziehungsweise 0,5 bis 5 Sekunden.

Als Reaktor kann zum Beispiel ein Rohrofen oder ein Rohrbündelreaktor verwendet werden, wie zum Beispiel ein Gegenstrom-Rohrofen mit Rauchgas als Wärmeträger oder ein Rohrbündelreaktor mit Salzschmelze als Wärmeträger. Das Propan zu Sauerstoff-Verhältnis im Ausgangsgemisch beträgt vorzugsweise 0,5:1 bis 40:1, insbesondere zwischen 1:1 bis 6:1, stärker bevorzugt zwischen 2:1 bis 5:1. Das Ausgangsgemisch kann auch weitere, im wesentlichen inerte, Bestandteile, wie Wasser, Kohlendioxid, Kohlenmonoxid, Stickstoff, Edelgase und/oder Propen umfassen, wobei es sich hierbei auch um zurückgeführte Bestandteile handeln kann.

Gestaltet man die Propandehydrierung als eine heterogen katalysierte Oxidehydrierung, so läßt sich diese prinzipiell durchführen wie beschrieben zum Beispiel in den Schriften US-A 4 788 371, CN-A 1073893, Catalysis Letters 23 (1994), 103-106, W. Zhang, Gaodeng Xuexiao Huaxue Xuebao, 14 (1993) 566, Z. Huang, Shiyou Huagong, 21 (1992) 592, WO 97/36849, DE-A 197 53 817, US-A 3 862 256, US-A 3 887 631, DE-A 195 30 454, US-A 4 341 664, J. of Catalysis 167, 560-569 (1997), J. of Catalysis 167, 550-559 (1997), Topics in Catalysis 3 (1996) 265-275, US-A 5 086 032, Catalysis Letters 10 (1991), 181-192, Ind. Eng. Chem. Res. 1996, 35, 14-18, US-A 4 255 284, Applied Catalysis A: General, 100 (1993), 111-130, J. of Catalysis 148, 56-67 (1994), V. Cortés Corberán und S. Vic Bellön (Ed.), New Developments in Selective Oxidation II, 1994, Elsevier Science B.V., S. 305-313, 3rd World Congress on Oxidation Catalysis, R.K. Grasselli, S.T. Oyama, A.M. Gaffney and J.E. Lyons (Ed.), 1997, Elsevier Science B.V., S. 375 ff.. Dabei kann als Sauerstoffquelle auch Luft eingesetzt werden. Häufig weist jedoch die Sauerstoffquelle hier zu mindestens 90 mol-% an molekularem Sauerstoff, und vielfach wenigsten 95 mol-% Sauerstoff auf.

Die für die heterogen katalysierte Oxidehydrierung geeigneten Katalysatoren unterliegen keinen besonderen Beschränkungen. Es eignen sich alle dem Fachmann auf diesem Gebiet bekannten Oxidehydrierungskatalysatoren, die in der Lage sind, Propan zu Propen zu oxidieren. Insbesondere können alle in den zuvor genannten Schriften genannten Oxidehydrierungskatalysatoren eingesetzt werden. Geeignete Katalysatoren sind beispielsweise Oxidehydrierungskatalysatoren, die MoVNb-Oxide oder Vanadylpyrophosphat, jeweils mit Promotor, umfassen. Ein Beispiel für einen günstigen Oxidehydrierkatalysator ist ein Katalysator, der ein Mischmetalloxid mit Mo, V, Te, O und X als wesentliche Bestandteile enthält, wobei X mindestens ein Element ist, ausgewählt aus Niob, Tantal, Wolfram, Titan, Aluminium, Zirkonium, Chrom, Mangan, Eisen, Ruthenium, Kobalt, Rhodium, Nickel, Palladium, Platin, Antimon, Bismut, Bor, Indium und Cer. Weiterhin besonders geeignete Oxidehydrierungskatalysatoren sind die Multimetalloxidmassen beziehungsweise -katalysatoren A der DE-A-197 53 817, wobei die in der vorgenannten Schrift als bevorzugt genannten Multimetalloxidmassen beziehungsweise -katalysatoren A ganz besonders günstig sind. Das heißt, als Aktivmassen kommen insbesondere Multimetalloxidmassen (IV) der allgemeinen Formel IV

M¹ₐMo_{1-b}M²_{b}Oₓ (IV),

wobei
M¹ = Co, Ni, Mg, Zn, Mn und/oder Cu,
M² = W, V, Te, Nb, P, Cr, Fe, Sb, Ce, Sn und/oder La,
a = 0,5-1,5,
b = 0-0,5
sowie
x = eine Zahl, die durch die Wertigkeit und Häufigkeit der von Sauerstoff verschiedenen Elemente in (IV) bestimmt wird,
in Betracht.

Prinzipiell können geeignete Aktivmassen (IV) in einfacher Weise dadurch hergestellt werden, daß man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 450 bis 1000°C calciniert. Als Quellen für die elementaren Konstituenten_ der Multimetalloxid-Aktivmassen (IV) kommen solche Verbindungen in Betracht, bei denen es sich bereits um Oxide handelt und/oder solche Verbindungen, die durch Erhitzen, wenigstens in Anwesenheit von Sauerstoff, in Oxide überführbar sind. Hierbei handelt es sich vom allem um Halogenide, Nitrate, Formiate, Oxalate, Citrate, Acetate, Carbonate, Amminkomplexsalze, Ammoniumsalze und/oder Hydroxide. Das innige Vermischen der Ausgangsverbindungen zur Herstellung der Multimetalloxidmassen (IV) kann in trockener Form, zum Beispiel als feinteiliges Pulver, oder in nasser Form, zum Beispiel mit Wasser als Lösungmittel, erfolgen. Die Multimetalloxidmassen (IV) können sowohl in Pulverform als auch zu bestimmten Katalysatorgeometrien geformt, eingesetzt werden, wobei die Formgebung vor oder nach der abschließenden Calcinierung erfolgen kann. Es können Vollkatalysatoren eingesetzt werden. Die Formgebung einer pulverförmigen Aktivmasse beziehungsweise Vorläufermasse kann aber auch durch Aufbringung auf vorgeformte inerte Katalysatorträger erfolgen. Als Trägermaterialien können dabei übliche, poröse oder unporöse Aluminiumoxide, Siliciumdioxid, Thoriumdioxid, Zirkondioxid, Siliciumcarbid oder Silikate verwendet werden, wobei die Trägerkörper regelmäßig oder unregelmäßig geformt sein können.

Für die heterogen katalysierte Oxidehydrierung des Propans liegt die Reaktionstemperatur vorzugsweise im Bereich von 200 bis 600°C, insbesondere im Bereich von 250 bis 500°C, stärker bevorzugt im Bereich von 350 bis 440°C. Der Arbeitsdruck liegt vorzugsweise im Bereich von 0,5 bis 10 bar, insbesondere von 1 bis 10 bar, stärker bevorzugt von 1 bis 5 bar. Arbeitsdrucke oberhalb von 1 bar, zum Beispiel von 1,5 bis 10 bar, haben sich als besonders vorteilhaft erwiesen. In der Regel erfolgt die heterogen katalysierte Oxidehydrierung des Propans an einem Katalysatorfestbett. Letzteres wird zweckmäßigerweise in den Rohren eines Rohrbündelreaktors aufgeschüttet, wie sie zum Beispiel in der EP-A-0 700 893 und in der EP-A-0 700 714 sowie der in diesen Schriften zitierten Literatur beschrieben sind. Die mittlere Verweilzeit des Reaktionsgasgemisches in der Katalysatorschüttung liegt zweckmäßigerweise bei 0,5 bis 20 Sekunden. Das Verhältnis von Propan zu Sauerstoff variiert mit dem gewünschten Umsatz und der Selektivität des Katalysators, zweckmäßigerweise liegt es im Bereich von 0,5:1 bis 40:1, insbesondere von 1:1 bis 6:1, stärker bevorzugt von 2:1 bis 5:1. In der Regel nimmt die Propenselektivität mit steigendem Propanumsatz ab. Vorzugsweise wird deshalb die Propan zu Propen-Reaktion so durchgeführt, daß relativ niedrige Umsätze mit Propan bei hohen Selektivitäten zu Propen erreicht werden. Besonders bevorzugt liegt der Umsatz an Propan im Bereich von 5 bis 40 mol-%, häufig im Bereich von 10 bis 30 mol-%. Hierbei bedeutet der Begriff "Propanumsatz" den Anteil an zugeführtem Propan, das beim einfacher Durchgang umgesetzt wird. In der Regel beträgt die Selektivität der Propanbildung 50 bis 98 mol-%, stärker bevorzugt 80 bis 98 mol-%, wobei der Begriff "Selektivität" die Mole an Propen bezeichnet, die pro Mol umgesetztem Propan erzeugt werden, ausgedrückt als molarer Prozentsatz.

In der Regel enthält das bei der oxidativen Propandehydrierung eingesetzte Ausgangsgemisch 5 bis 95 mol-% Propan (bezogen auf 100 mol-% Ausgangsgemisch). Neben Propan und Sauerstoff kann das Ausgangsgemisch für die heterogen katalysierte Oxidehydrierung auch weitere, insbesondere inerte, Bestandteile wie Kohlendioxid, Kohlenmonoxid, Stickstoff, Edelgase und/oder Propen umfassen. Die heterogene Oxidehydrierung kann auch in Anwesenheit von Verdünnungsmitteln, wie zum Beispiel Wasserdampf, durchgeführt werden.

Jede beliebige Reaktorsequenz kann zur Durchführung der homogenen Oxidehydrierung oder der heterogen katalysierten Oxidehydrierung des Propans eingesetzt werden, die dem Fachmann bekannt ist. Zum Beispiel kann die Oxidehydrierung in einem einzigen Reakor oder in einer Kaskade aus zwei oder mehreren Reaktoren, zwischen denen gegebenenfalls Sauerstoff eingeführt wird, durchgeführt werden. Es besteht auch die Möglichkeit, die homogene und die heterogen katalysierte Oxidehydrierung miteinander kombiniert zu praktizieren.

Als mögliche Bestandteile kann das Produktgemisch einer Propanoxidehydrierung zum Beispiel folgende Komponenten enthalten: Propen, Propan, Kohlendioxid, Kohlenmonoxid, Wasser, Stickstoff, Sauerstoff, Ethan, Ethen, Methan, Acrolein, Acrylsäure, Ethylenoxid, Butan, Essigsäure, Formaldehyd, Ameisensäure, Propylenoxid und Buten. In typischer Weise enthält ein bei der Propanoxidehydrierung erhaltenes Produktgemisch: 5 bis 10 mol-% Propen, 1 bis 2 mol-% Kohlenmonoxid, 1 bis 3 mol-% Kohlendioxid, 4 bis 10 mol.-% Wasser, 0 bis 1 mol.-% Stickstoff, 0,1 bis 0,5 mol-% Acrolein, 0 bis 1 mol.-% Acrylsäure, 0,05 bis 0,2 mol-% Essigsäure, 0,01 bis 0,05 mol-% Formaldehyd, 1 bis 5 mol-% Sauerstoff, 0,1 bis 1,0 mol% weitere oben genannte Komponenten, sowie als Rest im wesentlichen Propan, jeweils bezogen auf 100 mol-% Produktgemisch.

Generell kann die Propandehydrierung in der Reaktionszone A auch als eine heterogen katalysierte Propandehydrierung unter weitgehendem Sauerstoffausschluß wie in der DE-A 33 13 573 beschrieben oder wie folgt durchgeführt werden.

Da die heterogen katalysierte Dehydrierreaktion unter Volumenzunahme abläuft, kann der Umsatz durch Erniedrigung des Partialdrucks der Produkte gesteigert werden. Dies läßt sich in einfacher Weise, zum Beispiel durch Dehydrierung bei vermindertem Druck und/oder durch Zumischen von im wesentlichen inerten Verdünnungsgasen, wie zum Beispiel Wasserdampf, erreichen, der für die Dehydrierreaktion im Normalfall ein Inertgas darstellt. Eine Verdünnung mit Wasserdampf bedingt als weiteren Vorteil in der Regel ein vermindertes Verkoken des verwendeten Katalysators, da der Wasserdampf mit gebildetem Koks nach dem Prinzip der Kohlevergasung reagiert. Außerdem kann Wasserdampf als Verdünnungsgas in der nachfolgenden Oxidations- und/oder Ammoxidationszone B (in dieser Schrift als Kurzbezeichnung auch Reaktionszone B) mitverwendet werden. Wasserdampf läßt sich aber auch in einfacher Weise teilweise oder vollständig aus dem Produktgemisch A abtrennen (zum Beispiel durch Kondensieren), was die Möglichkeit eröffnet, bei der Weiterverwendung des dabei erhältlichen Produktgemisches A' in der Reaktionszone B den Anteil des Verdünnungsgases N₂ zu erhöhen. Weitere für die heterogen katalysierte Propandehydrierung geeignete Verdünnungsmittel sind zum Beispiel CO, Methan, Ethan, CO₂, Stickstoff und Edelgase wie He, Ne und Ar. Alle genannten Verdünnungsmittel können entweder für sich oder in Form unterschiedlichster Gemische mitverwendet werden. Es ist von Vorteil, dass die genannten Verdünnungsmittel in der Regel auch in der Reaktionszone B geeignete Verdünnungsmittel sind. Generell sind sich in der jeweiligen Stufe inert verhaltende (das heißt zu weniger als 5 mol-%, bevorzugt zu weniger als 3 mol-% und noch besser zu weniger als 1 mol-% sich chemisch verändernde) Verdünnungsmittel bevorzugt. Prinzipiell kommen für die heterogen katalysierte Propandehydrierung alle im Stand der Technik bekannten Dehydrierkatalysatoren in Betracht. Sie lassen sich grob in zwei Gruppen unterteilen. Nämlich in solche, die oxidischer Natur sind (zum Beispiel Chromoxid und/oder Aluminiumoxid) und in solche, die aus wenigstens einem auf einem, in der Regel oxidischen, Träger abgeschiedenen, in der Regel vergleichsweise edlen, Metall (zum Beispiel Platin) bestehen.

Unter anderem können damit alle Dehydrierkatalysatoren eingesetzt werden, die in der WO 99/46039, der US-A 4 788 371, der EP-A-0 705 136, der WO 99/29420, der US-A 4 220 091, der US-A 5 430 220, der US-A 5 877 369, der EP-A-0 117 146, der DE-A 199 37 196, der DE-A 199 37 105 sowie der DE-A 199 37 107 empfohlen werden. Im besonderen können sowohl der Katalysator gemäß Beispiel 1, Beispiel 2, Beispiel 3, und Beispiel 4 der DE-A 199 37 107 eingesetzt werden.

Dabei handelt es sich um Dehydrierkatalysatoren, die 10 bis 99,9 Gew.-% Zirkondioxid, 0 bis 60 Gew.-% Aluminiumoxid, Siliciumdioxid und/oder Titandioxid und 0,1 bis 10 Gew.-% mindestens eines Elements der ersten oder zweiten Hauptgruppe, eines Elements der dritten Nebengruppe, eines Elements der achten Nebengruppe des Periodensystems der Elemente, Lanthan und/oder Zinn enthalten, mit der Maßgabe, dass die Summe der Gewichtsprozente 100 Gew.-% ergibt.

Zur Durchführung der heterogen katalysierten Propandehydrierung kommen prinzipiell alle im Stand der Technik bekannten Reaktortypen und Verfahrensvarianten in Betracht. Beschreibungen solcher Verfahrensvarianten enthalten zum Beispiel alle bezüglich der Dehydrierkatalysatoren zitierten Schriften des Standes der Technik.

Eine vergleichsweise ausführliche Beschreibung von erfindungsgemäß geeigneten Dehydrierverfahren enthält auch "Catalytica® Studies Division, Oxidative Dehydrogenation and Alternative Dehydrogenation Processes, Study Number 4192 OD, 1993, 430 Ferguson Drive, Mountain View, California, 94043-5272 U.S.A..

Charakteristisch für die partielle heterogen katalysierte Dehydrierung von Propan ist, dass sie endotherm verläuft. Das heißt, die für die Einstellung der erforderlichen Reaktionstemperatur benötigte Wärme (Energie) muß entweder dem Reaktionsgasausgangsgemisch vorab und/oder im Verlauf der heterogen katalysierten Dehydrierung zugeführt werden.

Ferner ist es insbesondere für heterogen katalysierte Dehydrierungen von Propan aufgrund der hohen benötigten Reaktionstemperaturen typisch, dass in geringen Mengen schwersiedende hochmolekulare organische Verbindungen, bis hin zum Kohlenstoff, gebildet werden, die sich auf der Katalysatoroberfläche abscheiden und selbige dadurch deaktivieren. Um diese nachteilige Begleiterscheinung zu minimieren, kann das zur heterogen katalysierten Dehydrierung bei erhöhter Temperatur über die Katalysatoroberfläche zu leitende Propan haltige Reaktionsgasgemisch mit Wasserdampf verdünnt werden. Sich abscheidender Kohlenstoff wird unter den so gegebenen Bedingungen nach dem Prinzip der Kohlevergasung teilweise oder vollständig eliminiert.

Eine andere Möglichkeit, abgeschiedene Kohlenstoffverbindungen zu beseitigen, besteht darin, den Dehydrierkatalysator von Zeit zu Zeit bei erhöhter Temperatur mit einem Sauerstoff enthaltenden Gas zu durchströmen und damit den abgeschiedenen Kohlenstoff quasi abzubrennen. Eine weitgehende Unterdrückung der Bildung von Kohlenstoffablagerungen ist aber auch dadurch möglich, dass man dem heterogen katalysiert zu dehydrierenden Propan, bevor es bei erhöhter Temperatur über den Dehydrierkatalysator geführt wird, molekularen Wasserstoff zusetzt.

Selbstverständlich besteht auch die Möglichkeit, dem heterogen katalysiert zu dehydrierenden Propan Wasserdampf und molekularen Wasserstoff im Gemisch zuzusetzen. Ein Zusatz von molekularem Wasserstoff zur heterogen katalysierten Dehydrierung von Propan mindert auch die unerwünschte Bildung von Allen und Acetylen als Nebenprodukten.

Eine geeignete Reaktorform für die heterogen katalysierte Propandehydrierung ist der Festbettrohr- beziehungsweise Rohrbündelreaktor. Das heißt, der Dehydrierkatalysator befindet sich in einem oder in einem Bündel von Reaktionsrohren als Festbett. Die Reaktionsrohre werden dadurch beheizt, dass im die Reaktionsrohre umgebenden Raum ein Gas, zum Beispiel ein Kohlenwasserstoff wie Methan, verbrannt wird. Günstig ist es, diese direkte Form der Kontaktrohrerwärmung lediglich auf den ersten etwa 20 bis 30% der Festbettschüttung anzuwenden und die verbleibende Schüttungslänge durch die im Rahmen der Verbrennung freigesetzte Strahlungswärme auf die erforderliche Reaktionstemperatur aufzuwärmen. Auf diesem weg ist eine annähernd isotherme Reaktionsführung erreichbar. Geeignete Reaktionsrohrinnendurchmesser betragen etwa 10 bis 15 cm. Ein typischer Dehydrierrohrbündelreaktor umfaßt 300 bis 1000 Reaktionsrohre. Die Temperatur im Reaktionsrohrinneren bewegt sich im Bereich von 300 bis 700°C, vorzugsweise im Bereich von 400 bis 700°C. Mit Vorteil wird das Reaktionsgasausgangsgemisch dem Rohrreaktor auf die Reaktionstemperatur vorerwärmt zugeführt. Häufig verläßt das Produktgasgemisch das Reaktionsrohr mit einer 50 bis 100°C tiefergelegenen Temperatur. Im Rahmen der vorgenannten Verfahrensweise ist die Verwendung von oxidischen Dehydrierkatalysatoren auf der Grundlage von Chrom- und/oder Aluminiumoxid zweckmäßig. Häufig wird man kein Verdünnungsgas mitverwenden, sondern von im wesentlichen reinem Propan als Ausgangsreaktionsgas ausgehen. Auch der Dehydrierkatalysator wird meist unverdünnt angewandt.

Großtechnisch kann man mehrere (z.B. drei) solcher Rohrbündelreaktoren parallel betreiben. Dabei können sich erfindungsgemäß gegebenenfalls zwei dieser Reaktoren im Dehydrierbetrieb befinden, während in einem dritten Reaktor die Katalysatorbeschickung regeneriert wird, ohne dass der Betrieb in der Reaktionszone B leidet.

Eine solche Verfahrensweise ist beispielsweise bei dem in der Literatur bekannten BASF-Linde Propan-Dehydrierverfahren zweckmäßig. Erfindungsgemäß ist es aber von Bedeutung, dass die Verwendung eines solchen Rohrbündelreaktors ausreichend ist.

Eine solche Verfahrensweise ist auch beim sog. "steam active reforming (STAR) process" anwendbar, der von der Phillips Petroleum Co. entwickelt wurde (vergleiche zum Beispiel US-A 4 902 849, US-A 4 996 387 und US-A 5 389 342). Als Dehydrierkatalysator wird im STAR-Prozess mit Vorteil Promotoren enthaltendes Platin auf Zink (Magnesium) Spinel als Träger angewendet (vergleiche zum Beispiel US-A 5 073 662). Im Unterschied zum BASF-Linde Propan-Dehydrierverfahren wird das zu dehydrierende Propan beim STAR-Prozess mit Wasserdampf verdünnt. Typisch ist ein molares Verhältnis von Wasserdampf zu Propan im Bereich von 4 bis 6. Der Arbeitsdruck liegt häufig bei 3 bis 8 atm und die Reaktionstemperatur wird zweckmäßig zu 480 bis 620°C gewählt. Typische Katalysatorbelastungen mit dem totalen Reaktionsgasgemisch liegen bei 0,5 bis 10 h⁻¹ (LHSV).

Die heterogen katalysierte Propandehydrierung kann auch im Wanderbett gestaltet werden. Beispielsweise kann das Katalysatorwanderbett in einem Radialstromreaktor untergebracht sein. In selbigem bewegt sich der Katalysator langsam von oben nach unten, während das Reaktionsgasgemisch radial fließt. Diese Verfahrensweise wird beispielsweise im sogenannten UOP-Oleflex-Dehydrierverfahren angewandt. Da die Reaktoren bei diesem Verfahren quasi adiabat betrieben werden, ist es zweckmäßig, mehrere Reaktoren als Kaskade hintereinander geschaltet zu betreiben (in typischer Weise bis zu vier). Dadurch lassen sich zu hohe Unterschiede der Temperaturen des Reaktionsgasgemisches am Reaktoreingang und am Reaktorausgang vermeiden (bei der adiabaten Betriebsweise fungiert das Reaktionsgasausgangsgemisch als Wärmeträger, von dessen Wärmeinhalt der Abfall der Reaktionstemperatur abhängig ist) und trotzdem ansprechende Gesamtumsätze erzielen.

Wenn das Katalysatorbett den Wanderbettreaktor verlassen hat, wird es der Regenerierung zugeführt und anschließend wiederverwendet. Als Dehydrierkatalysator kann für dieses Verfahren zum Beispiel ein kugelförmiger Dehydrierkatalysator eingesetzt werden, der im wesentlichen aus Platin auf kugelförmigem Aluminiumoxidträger besteht. Bei der UOP-Variante wird dem zu dehydrierenden Propan Wasserstoff zugefügt, um eine vorzeitige Katalysatoralterung zu vermeiden. Der Arbeitsdruck liegt typisch bei 2 bis 5 atm. Das Wasserstoff zu Propan-Verhältnis (das molare) beträgt zweckmäßig 0,1 bis 1. Die Reaktionstemperaturen liegen bevorzugt bei 550 bis 650°C und die Kontaktzeit des Katalysators mit Reaktionsgasgemisch wird zu etwa 2 bis 6 h⁻¹ gewählt.

Bei den beschriebenen Festbettverfahren kann die Katalysatorgeometrie ebenfalls kugelförmig, aber auch zylindrisch (hohl oder voll) oder anderweitig geometrisch gestaltet sein.

Als weitere Verfahrensvariante für die heterogen katalysierte Propandehydrierung beschreibt Proceedings De Witt, Petrochem. Review, Houston, Texas, 1992 a, N1 die Möglichkeit einer heterogen katalysierten Propandehydrierung im Wirbelbett, bei der das Propan nicht verdünnt wird.

Erfindungsgemäß können dabei z.B. zwei Wirbelbetten nebeneinander betrieben werden, von denen sich eines ohne negative Auswirkungen auf den Gesamtprozess zeitweise im Zustand der Regenerierung befinden kann. Als Aktivmasse kommt dabei Chromoxid auf Aluminiumoxid zum Einsatz. Der Arbeitsdruck beträgt typisch 1 bis 1,5 atm und die Dehydriertemperatur liegt in der Regel bei 550 bis 600°C. Die für die Dehydrierung erforderliche Wärme wird dadurch in das Reaktionssystem eingebracht, dass der Dehydrierkatalysator auf die Reaktionstemperatur vorerhitzt wird. Der Arbeitsdruck liegt regelmäßig bei 1 bis 2 atm und die Reaktionstemperatur beträgt typisch 550 bis 600°C. Die vorstehende Dehydrierweise ist in der Literatur auch als Snamprogetti-Yarsintez Verfahren bekannt.

Alternativ zu den vorstehend beschriebenen Verfahrensweisen kann die heterogen katalysierte Propandehydrierung unter weitgehendem Sauerstoffausschluß auch nach einem von ABB Lummus Crest entwikkelten Verfahren realisiert werden (vergleiche Proceedings De Witt, Petrochem. Review, Houston, Texas, 1992, P1).

Den bisher beschriebenen heterogen katalysierten Dehydrierverfahren des Propans unter weitgehendem Sauerstoffausschluß ist gemein, dass sie bei Propanumsätzen von > 30 mol-% (in der Regel ≤ 60 mol-%) betrieben werden (bezogen auf einmaligen Reaktionszonendurchgang). Erfindungsgemäß von Vorteil ist es, dass es ausreichend ist, einen Propanumsatz von ≥ 5 mol-% bis ≤ 30 mol-% oder ≤ 25 mol-%, zu erzielen. Das heißt, die heterogen katalysierte Propandehydrierung kann auch bei Propanumsätzen von 10 bis 20 mol-% betrieben werden (die Umsätze beziehen sich auf einmaligen Reaktionszonendurchgang). Dies rührt unter anderem daher, dass die verbliebene Menge an nicht umgesetztem Propan in der nachfolgenden Reaktionszone B als inertes Verdünnungsgas fungiert und später weitgehend verlustfrei in die Reaktionszone rückgeführt werden kann.

Für die Realisierung der vorgenannten Propanumsätze ist es günstig, die heterogen katalysierte Propandehydrierung bei einem Arbeitsdruck von 0,3 bis 3 atm durchzuführen. Ferner ist es günstig, das heterogen katalytisch zu dehydrierende Propan mit Wasserdampf zu verdünnen. So ermöglicht die Wärmekapazität des Wassers einerseits, einen Teil der Auswirkung der Endothermie der Dehydrierung auszugleichen und andererseits reduziert die Verdünnung mit Wasserdampf den Edukt- und Produktpartialdruck, was sich günstig auf die Gleichgewichtslage der Dehydrierung auswirkt. Ferner wirkt sich die Wasserdampfmitverwendung, wie bereits erwähnt, vorteilhaft auf die Standzeit des Dehydrierkatalysators aus. Bei Bedarf kann als weiterer Bestandteil auch molekularer Wasserstoff zugegeben werden. Das molare Verhältnis von molekularem Wasserstoff zu Propan ist dabei in der Regel ≤ 5. Das molare Verhältnis von Wasserdampf zu Propan kann demnach mit vergleichsweise geringem Propanumsatz ≥ 0 bis 30, zweckmäßig 0,1 bis 2 und günstig 0,5 bis 1 betragen. Als günstig für eine Verfahrensweise mit niederem Propanumsatz erweist es sich auch, dass bei einmaligem Reaktordurchgang des Reaktionsgases lediglich eine vergleichsweise niedrige Wärmemenge verbraucht wird und zur Umsatzerzielung bei einmaligem Reaktordurchgang vergleichsweise niedrige Reaktionstemperaturen ausreichend sind.

Es kann daher zweckmäßig sein, die Propandehydrierung mit vergleichsweise geringem Propanumsatz (quasi) adiabat durchzuführen. Das heißt, man wird das Reaktionsgasausgangsgemisch in der Regel zunächst auf eine Temperatur von 500 bis 700°C (beziehungsweise von 550 bis 650°C) erhitzen (zum Beispiel durch Direktbefeuerung der es umgebenden Wandung). Im Normalfall wird dann ein einziger adiabater Durchgang durch ein Katalysatorbett ausreichend sein, um den gewünschten Umsatz zu erzielen, wobei sich das Reaktionsgasgemisch um etwa 30°C bis 200°C (je nach Umsatz) abkühlen wird. Ein Beisein von Wasserdampf als Wärmeträger macht sich auch unter dem Gesichtspunkt einer adiabaten Fahrweise vorteilhaft bemerkbar. Die niedrigere Reaktionstemperatur ermöglicht längere Standzeiten des verwendeten Katalysatorbetts.

Prinzipiell ist die heterogen katalysierte Propandehydrierung mit vergleichsweise geringem Propanumsatz, ob adiabat oder isotherm gefahren, sowohl in einem Festbettreaktor als auch in einem Wanderbett- oder Wirbelbettreaktor durchführbar.

Bemerkenswerterweise ist beim erfindungsgemäßen Verfahren zu ihrer Realisierung, insbesondere im adiabatischen Betrieb, ein einzelner Schachtofenreaktor als Festbettreaktor ausreichend, der vom Reaktionsgasgemisch axial und/oder radial durchströmt wird.

Im einfachsten Fall handelt es sich dabei um ein einziges geschlossenes Reaktionsvolumen, zum Beispiel einen Behälter, dessen Innendurchmesser 0,1 bis 10 m, eventuell auch 0,5 bis 5 m beträgt und in welchem das Katalysatorfestbett auf einer Trägervorrichtung (zum Beispiel ein Gitterrost) aufgebracht ist. Das mit Katalysator beschickte Reaktionsvolumen, das im adiabaten Betrieb wärmeisoliert ist, wird dabei mit dem heißen, Propan enthaltenden, Reaktionsgas axial durchströmt. Die Katalysatorgeometrie kann dabei sowohl kugelförmig als auch ringförmig oder strangförmig sein. Da in diesem Fall das Reaktionsvolumen durch einen sehr kostengünstigen Apparat zu realisieren ist, sind alle Katalysatorgeometrien, die einen besonders niedrigen Druckverlust aufweisen, zu bevorzugen. Das sind vor allem Katalysatorgeometrien, die zu einem großen Hohlraumvolumen führen oder strukturiert aufgebaut sind, wie zum Beispiel Monolithe bzw. Wabenkörper. Zur Verwirklichung einer radialen Strömung des Propan enthaltenden Reaktionsgases kann der Reaktor zum Beispiel aus zwei in einer Mantelhülle befindlichen, zentrisch ineinander gestellten, zylindrischen Gitterrosten bestehen und die Katalysatorschüttung in deren Ringspalt angeordnet sein. Im adiabaten Fall wäre die Metallhülle wiederum thermisch isoliert.

Als Katalysatorbeschickung für eine heterogen katalysierte Propandehydrierung mit vergleichsweise geringem Propanumsatz bei einmaligem Durchgang eignen sich insbesondere die in der DE-A 199 37 107 offenbarten, vor allem alle beispielhaft offenbarten, Katalysatoren.

Nach längerer Betriebsdauer sind vorgenannte Katalysatoren zum Beispiel in einfacher Weise dadurch regenerierbar, dass man bei einer Temperatur von 300 bis 600°C, häufig bei 400 bis 500°C, zunächst in ersten Regenerierungsstufen mit Stickstoff verdünnte Luft über das Katalysatorbett leitet. Die Katalysatorbelastung mit Regeneriergas kann dabei z.B. 50 bis 10000 h⁻¹ und der Sauerstoffgehalt des Regeneriergases 0,5 bis 20 Vol.-% betragen.

In nachfolgenden weiteren Regenerierungsstufen kann unter ansonsten gleichen Regenerierbedingungen als Regeneriergas Luft verwendet werden. Anwendungstechnisch zweckmäßig empfiehlt es sich, den Katalysator vor seiner Regenerierung mit Inertgas (zum Beispiel N₂) zu spülen.

Anschließend ist es in der Regel empfehlenswert, noch mit reinem molekularen Wasserstoff oder mit durch Inertgas verdünntem molekularem Wasserstoff (der Wasserstoffgehalt sollte ≥ 1 Vol.-% betragen) im ansonsten gleichen Bedingungsraster zu regenerieren.

Die heterogen katalysierte Propandehydrierung mit vergleichsweise geringem Propanumsatz (≤ 30 mol-%) kann in allen Fällen bei den gleichen Katalysatorbelastungen (sowohl das Reaktionsgas insgesamt, als auch das in selbigem enthaltende Propan betreffend) betrieben werden wie die Varianten mit hohem Propanumsatz (> 30 mol-%). Diese Belastung mit Reaktionsgas kann zum Beispiel 100 bis 10000 h⁻¹, häufig 100 bis 3000 h⁻¹, das heißt vielfach etwa 100 bis 2000 h⁻¹ betragen.

In besonders eleganter Weise läßt sich die heterogen katalysierte Propandehydrierung mit vergleichsweise geringem Propanumsatz in einem Hordenreaktor verwirklichen.

Dieser enthält räumlich aufeinanderfolgend mehr als ein die Dehydrierung katalysierendes Katalysatorbett. Die Katalysatorbettanzahl kann 1 bis 20, zweckmäßig 2 bis 8, aber auch 3 bis 6 betragen. Die Katalysatorbetten sind vorzugsweise radial oder axial hintereinander angeordnet. Anwendungstechnisch zweckmäßig wird in einem solchen Hordenreaktor der Katalysatorfestbetttyp angewendet.

Im einfachsten Fall sind die Katalysatorfestbetten in einem Schachtofenreaktor axial oder in den Ringspalten von zentrisch ineinandergestellten zylindrischen Gitterrosten angeordnet. Es ist jedoch auch möglich, die Ringspalte in Segmenten übereinander anzuordnen und das Gas nach radialem Durchtritt in einem Segment in das nächste darüber- oder darunterliegende Segment zu führen.

In zweckmäßiger Weise wird das Reaktionsgasgemisch auf seinem Weg von einem Katalysatorbett zum nächsten Katalysatorbett, zum Beispiel durch Überleiten mit heißen Gasen erhitzte Wärmetauscherrippen oder durch Leiten durch mit heißen Brenngasen erhitzte Rohre, im Hordenreaktor einer Zwischenerhitzung unterworfen.

Wird der Hordenreaktor im übrigen adiabat betrieben, ist es für die gewünschten Propanumsätze (≤ 30 mol-%) insbesondere bei Verwendung der in der DE-A 199 37 107 beschriebenen Katalysatoren, insbesondere der beispielhaften Ausführungsformen, ausreichend, das Reaktionsgasgemisch auf eine Temperatur von 450 bis 550°C vorerhitzt in den Dehydrierreaktor zu führen und innerhalb des Hordenreaktors in diesem Temperaturbereich zu halten. Das heißt, die gesamte Propandehydrierung ist so bei äußerst niederen Temperaturen zu verwirklichen, was sich für die Standzeit der Katalysatorfestbetten zwischen zwei Regenerierungen als besonders günstig erweist.

Noch geschickter ist es, die vorstehend geschilderte Zwischenerhitzung auf direktem Weg durchzuführen (autotherme Fahrweise). Dazu wird dem Reaktionsgasgemisch entweder bereits vor Durchströmung des ersten Katalysatorbettes und/oder zwischen den nachfolgenden Katalysatorbetten in begrenztem Umfang molekularer Sauerstoff zugesetzt. Je nach verwendetem Dehydrierkatalysator wird so eine begrenzte Verbrennung der im Reaktionsgasgemisch enthaltenen Kohlenwasserstoffe, gegebenenfalls bereits auf der Katalysatoroberfläche abgeschiedener Kohle beziehungsweise kohleähnlicher Verbindungen und/oder von im Verlauf der heterogen katalysierten Propandehydrierung gebildetem und/oder dem Reaktionsgasgemisch zugesetztem Wasserstoff bewirkt (es kann auch anwendungstechnisch zweckmäßig sein, im Hordenreaktor Katalysatorbetten einzufügen, die mit Katalysator beschickt sind, der spezifisch (selektiv) die Verbrennung von Wasserstoff (und/oder von Kohlenwasserstoff) katalysiert (als solche Katalysatoren kommen zum Beispiel jene der Schriften US-A 4 788 371, US-A 4 886 928, US-A 5 430 209, US-A 5 530 171, US-A 5 527 979 und US-A 5 563 314 in Betracht; beispielsweise können solche Katalysatorbetten in alternierender Weise zu den Dehydrierkatalysator enthaltenden Betten im Hordenreaktor untergebracht sein). Die dabei freigesetzte Reaktionswärme ermöglicht so auf quasi autotherme weise eine nahezu isotherme Betriebsweise der heterogen katalysierten Propandehydrierung. Mit zunehmender gewählter Verweilzeit des Reaktionsgases im Katalysatorbett ist so eine Propandehydrierung bei abnehmender oder im wesentlichen konstanter Temperatur möglich, was besonders lange Standzeiten zwischen zwei Regenerierungen ermöglicht.

In der Regel sollte eine wie vorstehend beschriebene Sauerstoffeinspeisung so vorgenommen werden, dass der Sauerstoffgehalt des Reaktionsgasgemisches, bezogen auf die darin enthaltene Menge an Propan und Propen, 0,5 bis 30 Vol.-% beträgt. Als Sauerstoffquelle kommen dabei sowohl reiner molekularer Sauerstoff oder mit Inertgas, zum Beispiel CO, CO₂, N₂, Edelgase, verdünnter Sauerstoff, insbesondere aber auch Luft, in Betracht. Die resultierenden Verbrennungsgase wirken in der Regel zusätzlich verdünnend und fördern dadurch die heterogen katalysierte Propandehydrierung.

Die Isothermie der heterogen katalysierten Propandehydrierung läßt sich dadurch weiter verbessern, dass man im Hordenreaktor in den Räumen zwischen den Katalysatorbetten geschlossene, vor ihrer Füllung günstigerweise aber nicht notwendigerweise evakuierte, Einbauten (zum Beispiel rohrförmige), anbringt. Derartige Einbauten können auch ins jeweilige Katalysatorbett gestellt werden. Diese Einbauten enthalten geeignete Feststoffe oder Flüssigkeiten, die oberhalb einer bestimmten Temperatur verdampfen oder schmelzen und dabei Wärme verbrauchen und dort, wo diese Temperatur unterschritten wird, wieder kondensieren und dabei Wärme freisetzen.

Eine Möglichkeit, das Reaktionsgasausgangsgemisch für die heterogen katalysierte Propandehydrierung auf die benötigte Reaktionstemperatur zu erwärmen, besteht auch darin, einen Teil des darin enthaltenen Propans und/oder H₂ mittels molekularem Sauerstoff zu verbrennen (zum Beispiel an geeigneten spezifisch wirkenden Verbrennungskatalysatoren, zum Beispiel durch einfaches Überleiten und/oder Durchleiten) und mittels der so freigesetzten Verbrennungswärme die Erwärmung auf die gewünschte Reaktionstemperatur zu bewirken. Die resultierenden Verbrennungsprodukte, wie CO₂, H₂O sowie das den für die Verbrennung benötigten molekularen Sauerstoff gegebenenfalls begleitende N₂ bilden vorteilhaft inerte Verdünnungsgase.

Das im Rahmen der heterogen katalysierten Propandehydrierung gebildete Produktgasgemisch A enthält in der Regel Propan, Propen, molekularen Wasserstoff, N₂, H₂O, Methan, Ethan, Ethylen, CO und CO₂. Es wird sich in der Regel bei einem Druck von 0,3 bis 10 atm befinden und häufig eine Temperatur von 400 bis 500°C, in günstigen Fällen von 450 bis 500°C aufweisen.

Während die EP-A 117 146, die DE-A 3 313 573 und die US-A 3 161 670 empfehlen, das in der heterogen katalysierten Propandehydrierung gebildete Produktgasgemisch A als solches zur Beschickung der Reaktionszone B zu verwenden, empfiehlt die DE-A 10028582 aus dem Produktgasgemisch A vor seiner Weiterverwendung zur Beschickung der Reaktionszone B wenigstens eine Teilmenge des bei der heterogen katalysierten Propandehydrierung gebildeten Wasserstoff abzutrennen.

Dies kann z.B. dadurch erfolgen, dass man das Produktgasgemisch A, gegebenenfalls nachdem man es zuvor in einem indirekten Wärmetauscher abgekühlt hat (zweckmäßigerweise wird die dabei entnommene Wärme zum Erhitzen eines für das erfindungsgemäße Verfahren benötigten feed-Gases verwendet) über eine, in der Regel zu einem Rohr gestaltete, Membran leitet, die lediglich für den molekularen Wasserstoff durchlässig ist. Der so abgetrennte molekulare Wasserstoff kann bei Bedarf teilweise in die heterogen katalysierte Dehydrierung von Propan rückgeführt oder einer sonstigen Verwertung zugeführt werden. Im einfachsten Fall kann er in Brennstoffzellen verbrannt werden.

Alternativ kann eine partielle oder vollständige Wasserstoffabtrennung auch durch partielle Kondensation, Adsorption und/oder Rektifikation (vorzugsweise unter Druck) vorgenommen werden. Die Teil- oder Vollabtrennung des molekularen Wasserstoff aus dem Produktgasgemisch A kann beim erfindungsgemäßen Verfahren auch durch selektive (z.B. heterogen katalysierte) Verbrennung desselben mit molekularem Sauerstoff vorgenommen werden. Das sich dabei bildende Reaktionswasser kann entweder teilweise oder vollständig abgetrennt oder im Gasgemisch belassen werden, da es in der Reaktionszone B als inertes Verdünnungsgas zu fungieren vermag. Diesbezüglich geeignete Katalysatoren offenbaren beispielsweise die US-A 4 788 371, US-A 4 886 928, US-A 5 430 209, US-A 5530171, US-A 5 527979 und US-A 5 563314.

Die Selektivverbrennung des molekularen Wasserstoff kann auch bereits während der heterogen katalysierten Dehydrierung quasi in situ erfolgen, z.B. durch Oxidatoren mittels eines dem Dehydrierungskatalysator zusätzlich zugesetzten reduzierbaren Metalloxids, wie es z.B. die EP-A 832056 beschreibt.

Erfindungsgemäß zweckmäßig wird man wenigstens 10 mol-%, oder wenigstens 25 mol-%, häufig wenigstens 35 mol-%, oder wenigsten 50 mol-%, vielfach wenigstens 75 mol-% und oft die Gesamtmenge des im Rahmen der heterogen katalysierten Dehydrierung gebildeten molekularen Wasserstoff abtrennen, bevor das verbleibende Produktgasgemisch A' zur Beschickung der Reaktionszone B verwendet wird. Bei Bedarf kann aus dem Produktgasgemisch A vor seiner Weiterverarbeitung in der Reaktionszone B auch eventuell enthaltenes Wasser abgetrennt (z.B. Auskondensieren) werden. Selbstredend kann bei Bedarf im Rahmen der Abtrennung von molekularem Wasserstoff auch eine Abtrennung anderer, von Propan und Propylen verschiedener, Bestandteile des Produktgasgemisches A vorgenommen werden.

Eine einfache Möglichkeit, im wesentlichen alle von Propan und Propen verschiedenen Bestandteile des Produktgasgemisches A abzutrennen, besteht darin, das vorzugsweise abgekühlte (vorzugsweise auf Temperaturen von 10 bis 70°C), Produktgasgemisch A, z.B. bei einem Druck von 0,1 bis 50 atm und einer Temperatur von 0 bis 100°C, mit einem (vorzugsweise hochsiedenen) organischen Lösungsmittel (vorzugsweise ein hydrophobes), in welchem Propan und Propen bevorzugt absorbiert werden, in Kontakt zu bringen (z.B. durch einfaches Durchleiten). Durch nachfolgende Desorption, Rektifikation und/oder Strippung mit einem bezüglich der Reaktionszone B sich inert verhaltenden und/oder in dieser Reaktionszone als Reaktand benötigten Gas (z.B. Luft) werden das Propan und Propen im Gemisch in vergleichsweise reiner Form rückgewonnen und zur Beschichtung der Reaktionszone B verwendet. Das den molekularen Wasserstoff enthaltende Abgas der Absorption kann man z.B. wieder einer Membrantrennung unterwerfen und dann, bei Bedarf, den abgetrennten Wasserstoff bei der heterogen katalysierten Propandehydrierung mitverwenden.

Insbesondere die vorstehend beschriebene Abtrennung ist eine ausgezeichnete Basis dafür, dass für die heterogen katalysierte Propandehydrierung kein reines Propan eingesetzt werden muss. Vielmehr kann das verwendete Propan bis zu 50 Vol-% anderer Gase wie z.B. Ethan, Methan, Ethylen, Butane, Butene, Acetylen, H₂S, SO₂, Pentane etc. enthalten, wird der größte Teil dieser Nebenprodukte doch im Rahmen des beschriebenen Trennschrittes mitabgetrennt. Als Alternative für den beschriebenen Trennschritt via Absorption wäre auch eine Druckwechseladsorption denkbar. Zweckmäßig enthält das einzusetzende Rohpropan wenigstens 60 Vol.-%, vorteilhaft wenigstens 70 Vol.-%, bevorzugt wenigstens 80 Vol.-%, besonders bevorzugt wenigstens 90 Vol.-% und ganz besonders bevorzugt wenigstens 95 Vol-% an Propan. Insbesondere kann für die heterogen katalysierte Propandehydrierung auch ein Gemisch aus Propan, Propen und aus der Oxidationsstufe herrührendem Kreisgas verwendet werden.

Als Absorptionsmittel für die vorstehend beschriebene Abtrennung eignen sich grundsätzlich alle Absorptionsmittel, die in der Lage sind, Propan und Propen zu absorbieren. Bei dem Absorptionsmittel handelt es sich vorzugsweise um ein organisches Lösungsmittel, das vorzugsweise hydrophob und/oder hochsiedend ist. Vorteilhafterweise hat dieses Lösungsmittel einen Siedepunkt (bei einem Normaldruck von 1 atm) von mindestens 120°C, bevorzugt von mindestens 180 °C, vorzugsweise von 200 bis 350°C, insbesondere von 250 bis 300°C, stärker bevorzugt von 260 bis 290°C. Zweckmäßigerweise liegt der Flammpunkt (bei einem Normaldruck von 1 atm) über 110°C. Allgemein eignen sich als Absorptionsmittel relativ unpolare organische Lösungsmittel, wie zum Beispiel aliphatische Kohlenwasserstoffe, die vorzugsweise keine nach außen wirkende polare Gruppe enthalten, aber auch aromatische Kohlenwasserstoffe. Allgemein ist es erwünscht, dass das Absorptionsmittel einen möglichst hohen Siedepunkt bei gleichzeitig möglichst hoher Löslichkeit für Propan und Propen hat. Beispielhaft als Absorptionsmittel genannt seien aliphatische Kohlenwasserstoffe, zum Beispiel C₈-C₂₀-Alkane oder -Alkene, oder aromatische Kohlenwasserstoffe, zum Beispiel Mittelölfraktionen aus der Paraffindestillation oder Ether mit sperrigen Gruppen am O-Atom, oder Gemische davon, wobei diesen ein polares Lösungsmittel, wie zum Beispiel das in der DE-A 43 08 087 offenbarte 1,2-Dimethylphthalat zugesetzt sein kann. Weiterhin eignen sich Ester der Benzoesäure und Phthalsäure mit geradkettigen, 1 bis 8 Kohlenstoffatome enthaltenden Alkanolen, wie Benzoesäure-n-butylester, Benzoesäuremethylester, Benzoesäureethylester, Phthalsäuredimethylester, Phthalsäurediethylester, sowie sogenannte Wärmeträgeröle, wie Diphenyl, Diphenylether und Gemische aus Diphenyl und Diphenylether oder deren Chlorderivate und Triarylalkene, zum Beispiel 4-Methyl-4'-benzyl-diphenylmethan und dessen Isomere 2-Methyl-2'-benzyl-diphenyl-methan, 2-Methyl-4'-benzyldiphenylmethan und 4-Methyl-2'-benzyl-diphenylmethan und Gemische solcher Isomerer. Ein geeignetes Absorptionsmittel ist ein Lösungsmittelgemisch aus Diphenyl und Diphenylether, bevorzugt in der azeotropen Zusammensetzung, insbesondere aus etwa 25 Gew.-% Diphenyl (Biphenyl) und etwa 75 Gew.-% Diphenylether, beispielsweise das im Handel erhältliche Diphyl. Häufig enthält dieses Lösungsmittelgemisch ein Lösungsmittel wie Dimethylphthalat in einer Menge von 0,1 bis 25 Gew.-%, bezogen auf das gesamte Lösungsmittelgemisch, zugesetzt. Besonders geeignete Absorptionsmittel sind auch Octane, Nonane, Decane, Undecane, Dodecane, Tridecane, Tetradecane, Pentadecane, Hexadecane, Heptadecane und Octadecane, wobei sich insbesondere Tetradecane als besonders geeignet erwiesen haben. Günstig ist es, wenn das verwendete Absorptionsmittel einerseits den oben genannten Siedepunkt erfüllt, andererseits gleichzeitig aber ein nicht zu hohes Molekulargewicht aufweist. Mit Vorteil beträgt das Molekulargewicht des Absorptionsmittels = 300 g/mol. Geeignet sind auch die in DE-A 33 13 573 beschriebenen Paraffinöle mit 8 bis 6 Kohlenstoffatomen. Beispiele für geeignete Handelsprodukte sind von der Firma Haltermann vertriebene Produkte wie Halpasole i, wie Halpasol 250/340 i und Halpasol 250/275 i, sowie Druckfarbenöle unter den Bezeichnungen PKWF und Printosol.

Die Durchführung der Absorption unterliegt keinen besonderen Beschränkungen. Es können alle dem Fachmann gängigen Verfahren und Bedingungen eingesetzt werden. Vorzugsweise wird das Gasgemisch bei einem Druck von 1 bis 50 bar, bevorzugt 2 bis 20 bar, stärker bevorzugt 5 bis 10 bar, und einer Temperatur von 0 bis 100°C, insbesondere von 30 bis 50°C, mit dem Absorptionsmittel in Kontakt gebracht. Die Absorption kann sowohl in Kolonnen als auch in Quenchapparaten vorgenommen werden. Dabei kann im Gleichstrom oder im Gegenstrom gearbeitet werden. Geeignete Absorptionskolonnen sind zum Beispiel Bodenkolonnen (mit Glocken- und/oder Siebböden), Kolonnen mit strukturierten Packungen (zum Beispiel Blechpackungen mit einer spezifischen Oberfläche von 100 bis 500 m²/m³, zum Beispiel Mellapak^{®} 250 Y) und Füllkörperkolonnen (zum Beispiel mit Raschig-Füllkörpern gefüllte). Es können aber auch Riesel- und Sprühtürme, Graphitblockabsorber, Oberflächenabsorber wie Dickschicht- und Dünnschichtabsorber sowie Tellerwäscher, Kreuzschleierwäscher und Rotationswäscher verwendet werden. Zudem kann es günstig sein, die Absorption in einer Blasensäule mit und ohne Einbauten stattfinden zu lassen.

Die Abtrennung des Propans und Propens von dem Absorptionsmittel kann durch Strippung, Entspannungsverdampfung (Flashen) und/oder Destillation erfolgen.

Die Abtrennung des Propans und Propens von dem Absorptionsmittel erfolgt vorzugsweise durch Strippen und/oder Desorption. Die Desorption kann in üblicher Weise über eine Druck- und/oder Temperaturänderung durchgeführt werden, vorzugsweise bei einem Druck von 0,1 bis 10 bar, insbesondere 1 bis 5 bar, stärker bevorzugt 1 bis 3 bar, und einer Temperatur von 0 bis 200°C, insbesondere 20 bis 100°C, stärker bevorzugt 30 bis 50°C. Ein für die Strippung geeignetes Gas ist beispielsweise Wasserdampf, bevorzugt sind jedoch insbesondere Sauerstoff-/Stickstoff-Mischungen, beispielsweise Luft. Bei der Verwendung von Luft beziehungsweise Sauerstoff-/Stickstoff-Mischungen, bei denen der Sauerstoffgehalt über 10 Vol-% liegt, kann es sinnvoll sein, vor oder während des Strippprozesses ein Gas zuzusetzen, das den Explosionsbereich reduziert. Besonders geeignet dafür sind Gase mit einer spezifischen Wärmekapazität >29 J/mol·K bei 20°C, wie zum Beispiel Methan, Ethan, Propan, Propen, Butan, Pentan, Hexan, Benzol, Methanol, Ethanol, sowie Ammoniak, Kohlendioxid, und Wasser. Besonders geeignet für die Strippung sind auch Blasensäulen mit und ohne Einbauten.

Die Abtrennung des Propans und Propens von dem Absorptionsmittel kann auch über eine Destillation erfolgen, wobei die dem Fachmann geläufigen Kolonnen mit Packungen, Füllkörpern oder entsprechenden Einbauten verwendet werden können. Bevorzugte Bedingungen bei der Destillation sind ein Druck von 0,01 bis 5 bar, insbesondere 0,1 bis 4 bar, stärker bevorzugt 1 bis 3 bar, und eine Temperatur (im Sumpf) von 50 bis 300°C, insbesondere 150 bis 250°C.

Ein durch Strippen aus dem Absorptionsmittel gewonnenes Produktgasgemisch A' kann vor seiner Verwendung zur Beschickung der Reaktionszone B noch einer weiteren Verfahrensstufe zugeführt werden, um z.B. die Verluste an mitgestripptem Absorptionsmittel zu reduzieren und die Reaktionszone B so gleichzeitig vor Absorptionsmittel zu schützen. Eine solche Abtrennung des Absorptionsmittels kann nach allen dem Fachmann bekannten Verfahrensschritten erfolgen. Eine im Rahmen des erfindungsgemäßen Verfahrens bevorzugte Ausführungsform einer solchen Abtrennung ist z.B. das Quenchen des Ausgangsstromes aus der Strippvorrichtung mit Wasser. In diesem Fall wird das Absorptionsmittel aus diesem beladenen Ausgangsstrom mit Wasser herausgewaschen. Diese Wäsche beziehungsweise das Quenchen kann z.B. am Kopf einer Desorptionskolonne über einem Flüssigkeits-Fangboden durch Gegensprühen von Wasser oder in einem eigenen Apparat erfolgen.

Zur Unterstützung des Trenneffektes können im Quenchraum die Quenchoberfläche vergößernde Einbauten installiert werden, wie sie dem Fachmann von Rektifikationen, Absorptionen und Desorptionen her bekannt sind.

Wasser ist insofern ein bevorzugtes Waschmittel, da es in der nachfolgenden Reaktionszone B normalerweise nicht stört. Nachdem das Wasser das Absorptionsmittel aus dem mit Propan und Propen beladenen Ausgangstrom herausgewaschen hat, kann das Wasser/Absorptionsmittel-Gemisch einer Phasentrennung D zugeführt und der behandelte Ausgangstrom als ein Produktgasgemisch A' der Reaktionszone B zugeführt werden.

Sowohl das C₃-frei gestrippte Absorptionsmittel als auch das in der Phasentrennung rückgewonnene Absorptionsmittel können für den Absorptionszweck wiederverwendet werden.

Das Produktgasgemisch A oder das aus selbigem erzeugte Produktgasgemisch A' kann nun in an sich bekannter Weise zur Beschickung einer heterogen katalysierten Gasphasenoxidation von Propen zu Acrolein und/oder Acrylsäure mit einem Beschickungsgasgemisch A" eingesetzt werden. Als Oxidationsmittel kann dabei reiner molekularer Sauerstoff, Luft oder mit Sauerstoff angereicherte Luft zugesetzt werden.

Häufig wird man beim erfindungsgemäßen Verfahren die Zusammensetzung des Beschickungsgasgemisches A" unter Mitverwendung des Produktgasgemisch A' so einstellen, dass es die nachfolgenden molaren Verhältnisse erfüllt:
Propan : Propen : N₂ : O₂ : H₂O : sonstige
   = 0,5 bis 20 : 1 : 0,1 bis 40 : 0,1 bis 10 : 0 bis 20 : 0 bis 1.
Mit Vorteil betragen die vorgenannten molaren Verhältnisse erfindungsgemäß
   = 2 bis 10 : 1 : 0,5 bis 20 : 0,5 bis 5 : 0,01 bis 10 : 0 bis 1.
Günstig ist es auch, wenn die vorgenannten molaren Verhältnisse erfindungsgemäß
   = 3 bis 6 : 1 : 1 bis 10 : 1 bis 3 : 0,1 bis 2 : 0 bis 0,5 betragen.

Prinzipiell läuft die heterogen katalysierte Gasphasen-Partialoxidation von Propylen zu Acrylsäure mit molekularem Sauerstoff in zwei längs der Reaktionskoordinate aufeinanderfolgenden Schritten ab, von denen der erste zum Acrolein und der zweite vom Acrolein zur Acrylsäure führt.

Dieser Reaktionsablauf in zwei zeitlich aufeinanderfolgenden Schritten eröffnet in an sich bekannter Weise die Möglichkeit, die Reaktionszone B des erfindungsgemäßen Verfahrens in zwei hintereinander angeordneten Oxidationszonen auszuführen, wobei in jeder der beiden Oxidationszonen der zu verwendende oxidische Katalysator in optimierender Weise angepaßt werden kann. So wird für die erste Oxidationszone (Propylen → Acrolein) in der Regel ein Katalysator auf der Basis von die Elementkombination Mo-Bi-Fe enthaltenden Multimetalloxiden bevorzugt, während für die zweite Oxidationszone (Acrolein → Acrylsäure) normalerweise Katalysatoren auf der Basis von die Elementkombination Mo-V enthaltenden Multimetalloxiden bevorzugt werden.

Entsprechende Multimetalloxidkatalysatoren für die beiden Oxidationszonen sind vielfach vorbeschrieben und dem Fachmann wohlbekannt. Beispielsweise verweist die EP-A 253 409 auf Seite 5 auf entsprechende US-Patente.

Günstige Katalysatoren für die beiden Oxidationszonen offenbaren auch die DE-A 4 431 957 und die DE-A 4431949. Dieses gilt insbesondere für jene der allgemeinen Formel I in den beiden vorgenannten Schriften. In der Regel wird das Produktgemisch aus der ersten Oxidationszone ohne Zwischenbehandlung in die zweite Oxidationszone überführt.

Die einfachste Realisierungsform der beiden Oxidationszonen bildet daher ein Rohrbündelreaktor innerhalb dessen sich die Katalysatorbeschickung längs der einzelnen Kontaktrohre mit Beendigung des ersten Reaktionsschrittes entsprechend ändert (derartige als Reaktionszone B erfindungsgemäß geeignete Propylenpartialoxidationen lehren z.B. die EP-A 911313, die EP-A 979813, die EP-A 990636 und die DE-A 2830765). Gegebenenfalls wird dabei die Beschickung der Kontaktrohre mit Katalysator durch eine Inertschüttung unterbrochen.

Vorzugsweise werden die beiden Oxidationszonen jedoch in Form zweier hintereinander geschalteter Rohrbündelsysteme realisiert. Diese können sich in einem Reaktor befinden, wobei der Übergang von einem Rohrbündel zum anderen Rohrbündel von einer nicht im Kontaktrohr untergebrachten (zweckmäßigerweise begehbaren) Schüttung aus Inertmaterial gebildet werden. Während die Kontaktrohre in der Regel von einem Wärmeträger umspült werden, erreicht dieser eine wie vorstehend beschrieben angebrachte Inertschüttung nicht. Mit Vorteil werden daher die beiden Kontaktrohrbündel in räumlich voneinander getrennten Reaktoren untergebracht. In der Regel befindet sich dabei zwischen den beiden Rohrbündelreaktoren ein Zwischenkühler, um eine gegebenenfalls erfolgende Acroleinnachverbrennung im Produktgasgemisch, das die erste Oxidationsstufe verläßt, zu mindern. Anstelle von Rohrbündelreaktoren können auch Plattenwärmetauscherreaktoren mit Salz- und/oder Siedekühlung, wie sie z.B. die DE-A 19 929 487 und die DE-A 19 952 964 beschreiben, eingesetzt werden.

Die Reaktionsstemperatur in der ersten Oxidationszone liegt in der Regel bei 300 bis 450°C, bevorzugt bei 320 bis 390°C. Die Reaktionstemperatur in der zweiten Oxidationszone liegt in der Regel bei 200 bis 300°C, häufig bei 220 bis 290°C. Der Reaktionsdruck beträgt in beiden Oxidationszonen zweckmäßig 0,5 bis 5, vorteilhaft 1 bis 3 atm. Die Belastung (Nl/l·h) der Oxidationskatalysatoren mit Reaktionsgas beträgt in beiden Oxidationszonen häufig 1500 bis 2500 h⁻¹ bzw. bis 4000 h⁻¹. Die Belastung mit Popen kann dabei bei 100 bis 200 und mehr Nl/l·h liegen.

Prinzipiell können die beiden Oxidationszonen beim erfindungsgemäßen Verfahren so gestaltet werden, wie es z.B. in der DE-A 19 837 517, der DE-A 19 910 506, der DE-A 19 910 508 sowie der DE-A 19 837 519 beschrieben ist. Üblicherweise wird die externe Temperierung in den beiden Oxidationszonen, gegebenenfalls in Mehrzonenreaktorsystemen, in an sich bekannter Weise an die spezielle Reaktionsgasgemischzusammensetzung sowie Katalysatorbeschickung angepaßt.

Der für die erfindungsgemäß erforderliche Reaktionszone B als Oxidationsmittel insgesamt benötigte molekulare Sauerstoff kann dem Beschickungsgasgemisch der Reaktionszone B in seiner Gesamtmenge vorab zugegeben werden. Selbstverständlich kann aber auch nach der ersten Oxidationszone mit Sauerstoff ergänzt werden.

Vorzugsweise wird in der ersten Oxidationszone ein molares Verhältnis Propylen : molekularer Sauerstoff von 1 : 1 bis 3, häufig 1 : 1,5 bis 2 eingestellt. Ähnliche numerische Werte eignen sich für das molare Verhältnis Acrolein: molekularer Sauerstoff in der zweiten Oxidationszone (1 : 0,5 bis 1,5 wäre bevorzugt).

In beiden Oxidationszone wirkt sich ein Überschuß an molekularem Sauerstoff in der Regel vorteilhaft auf die Kinetik der Gasphasenoxidation aus. Im Unterschied zu den Verhältnissen in der erfindungsgemäß anzuwendenden Reaktionszone A werden die thermodynamischen Verhältnisse durch das molare Reaktandenverhältnis im wesentlichen nicht beeinflußt, da die heterogen katalysierte Gasphasen-Partialoxidation des Propylens zu Acrylsäure kinetischer Kontrolle unterliegt. Prinzipiell kann daher z.B. in der ersten Oxidationszone auch das Propylen gegenüber dem molekularen Sauerstoff im molaren Überschuß vorgelegt werden. In diesem Fall kommt dem überschüssigen Propylen faktisch die Rolle eines Verdünnungsgases zu.

Prinzipiell ist die heterogen katalysierte Gasphasen-Partialoxidation von Propylen zu Acrylsäure aber auch in einer einzigen Oxidationszone realisierbar. In diesem Fall erfolgen beide Reaktionsschritte in einem Oxidationsreaktor der mit einem Katalysator beschickt ist, der die Umsetzung beider Reaktionsschritte zu katalysieren vermag. Selbstredend kann sich auch die Katalysatorbeschickung innerhalb der Oxidationszone längs der Reaktionskoordinate kontinuierlich oder abrupt ändern. Natürlich kann bei einer Ausführungsform der erfindungsgemäß anzuwendenden Reaktionszone B in Gestalt zweier hintereinandergeschalteter Oxidationszonen aus dem die erste Oxidationszone verlassenden Produktgasgemisch in selbigem enthaltenes, in der ersten Oxidationszone als Nebenprodukt entstandenes, Kohlenoxid und Wasserdampf bei Bedarf vor der Weiterleitung in die zweite Oxidationszone teilweise oder vollständig abgetrennt werden. Vorzugsweise wird man erfindungsgemäß eine Verfahrensweise wählen, die solch einer Abtrennung nicht bedarf.

Als Quelle für den in der Reaktionszone B benötigten molekularen Sauerstoff, der dem Produktgasgemisch A bzw. A' vor dessen Verwendung zur Beschickung der Reaktionszone B zugemischt wird, kommen sowohl reiner molekularer Sauerstoff als auch mit Inertgas wie CO₂, CO, Edelgasen, N₂ und/oder gesättigten Kohlenwasserstoffen verdünnter molekularer Sauerstoff in Betracht.

In zweckmäßiger Weise wird man wenigstens zur Deckung eines Teilbedarfs an molekularem Sauerstoff Luft als Sauerstoffquelle verwenden.

Mit Vorteil wird beim erfindungsgemäßen Verfahren das Produktgasgemisch A' im wesentlichen nur aus Propan und Propylen bestehen (der Anteil an davon verschiedenen Bestandteilen beträgt zweckmäßig ≤ 5 Vol.-% bzw. ≤ 2 Vol.-%) und als Quelle für molekularen Sauerstoff für die nachfolgende Reaktionszone B wird ausschließlich Luft verwendet, die in der Regel komprimiert (typisch 2-3 bar) und dabei erwärmt (typisch 130°C - 180°C) zugegeben wird.

Durch Zudosieren von kalter Luft zu heißem Produktgasgemisch A' kann im Rahmen des erfindungsgemäßen Verfahrens auch auf direktem Weg eine Abkühlung des Produktgasgemisches A' bewirkt werden.

Ist Acrolein das Zielprodukt, wird man in der Reaktionszone B die zweite Oxidationszone in zweckmäßiger Weise nicht mehr anwenden.

Das die erfindungsgemäß anzuwendende Reaktionszone B verlassende Produktgasgemisch B ist in der Regel im wesentlichen zusammengesetzt aus dem Zielprodukt Acrolein oder Acrylsäure oder dessen Gemisch mit Acrolein, nicht umgesetztem molekularem Sauerstoff, Propan, nicht umgesetztem Propen, molekularem Stickstoff, als Nebenprodukt entstandenem und/oder als Verdünnungsgas mitverwendetem Wasserdampf; als Nebenprodukt und/oder als Verdünnungsgas mitverwendeten Kohlenoxiden sowie geringen Mengen sonstiger niederer Aldehyde, niederer Alkancarbonsäuren (z.B. Essigsäure, Ameisensäure und Propionsäure) sowie Maleinsäureanhydrid, Benzaldehyd, Kohlenwasserstoffe und anderer inerter Verdünnungsgase.

Das Zielprodukt kann aus dem Produktgasgemisch B in an sich bekannter Weise abgetrennt werden (z.B. durch partielle Kondensation der Acrylsäure oder durch Absorption von Acrylsäure in Wasser oder in einem hochsiedenden hydrophoben organischen Lösungsmittel oder durch Absorption von Acrolein in Wasser oder in wäßrigen Lösungen niederer Carbonsäuren sowie anschließende Aufarbeitung der Absorbate; alternativ kann das Produktgasgemisch B auch fraktioniert kondensiert werden; vgl. z.B. EP-A 117146, DE-A 4308087, DE-A 4335172, DE-A 4436243, DE-A 19 924 532 sowie DE-A 19 924 533).

Nicht umgesetztes Propylen und/oder Acrolein werden gegebenenfalls gleichfalls abgetrennt und in die Reaktionszone B rückgeführt.

Ansonsten können die von Acrylsäure und Acrolein verschiedenen wesentlichen Bestandteile des nach der Zielproduktabtrennung verbleibenden Restgases je nach Bedarf und verwendetem Dehydrierkatalysator jeweils für sich abgetrennt und/oder mit dem Propan als Kreisgas (Rückführstrom) in die Reaktionszone A rückgeführt werden, um dort, wie beschrieben, den Dehydrierumsatz zu beeinflussen. Selbstverständlich kann aber auch das nicht umgesetzte Propan im Gemisch mit dem nicht umgesetzten Propylen für sich (als Rückführstrom) in die Reaktionszone A rückgeführt werden. Bei kontinuierlicher Ausführung des erfindungsgemäßen Verfahrens erfolgt so eine kontinuierliche Umsetzung von Propan zu Acrylsäure und/oder Acrolein.

Die Abtrennung von Propan und Propen aus dem nach der Zielproduktabtrennung verbleibenden Restgas (es enthält in der Regel O₂, CO, CO₂, H₂O, N₂, Edelgase sowie sonstige niedere Aldehyde, niederer Alkancarbonsäuren (z.B. Essigsäure, Ameisensäure und Propionsäure) sowie Maleinsäureanhydrid, Benzaldehyd und Kohlenwasserstoffe) kann, wie bereits beschrieben, durch Absorption mit nachfolgender Desorption und/oder Strippung (sowie Absorptionsmittelwiederverwendung) in einem hochsiedenden hydrophoben organischen Lösungsmittel erfolgen. Weitere Trennmöglichkeiten sind Adsorption, Rektifikation und partielle Kondensation.

Bei Verwendung von Dehydrierkatalysatoren, die gegenüber Sauerstoff oder Sauerstoff enthaltenden Verbindungen empfindlich sind wird man diese Oxygenate vor einer Rückführung von Krelsgas in die Stufe A aus dem Kreisgas abtrennen. Eine solche Sauerstoffabtrennung kann auch sinnvoll sein um eine Oxidation des Propans in der Dehydrierstufe A zu vermeiden. Die Dehydrierkatalysatoren der DE-A 19 937 107 sind nicht empfindlich gegen Oxygenate (insbesondere jene gemäß Beispiel 1 bis 4 der DE-A).

Eine andere Abtrennmöglichkeit bietet, wie gleichfalls bereits erwähnt, die fraktionierte Destillation. Vorzugsweise wird eine fraktionierte Druckdestillation bei tiefen Temperaturen durchgeführt. Der anzuwendende Druck kann z.B. 10 bis 100 bar betragen.

Als Rektifikationskolonnen können Füllkörperkolonnen, Bodenkolonnen oder Packungskolonnen eingesetzt werden. Als Bodenkolonnen eignen sich solche mit Dual-Flow-Böden, Glockenböden oder Ventilböden. Das Rücklaufverhältnis kann z.B. 1 bis 10 betragen. Andere Trennmöglichkeiten bilden z.B. Druckextraktion, Druckwechseladsorption, Druckwäsche und partielle Kondensation.

Selbstverständlich kann erfindungsgemäß, z.B. dann, wenn nach der Reaktionszone A eine Abtrennung von Nebenkomponenten integriert ist, auch die Gesamtmenge an Restgas (als Rückführstrom) in die Reaktionszone A rückgeführt werden. In diesem Fall kann sich der Auslaß für von Propan, Propen und molekularem Sauerstoff verschiedene Gasbestandteile ausschließlich zwischen dem Produktgasgemisch A und dem Produktgasgemisch A' befinden.

Selbstredend kann ein weiterer Auslaß nach der Zielproduktabtrennung eingerichtet sein. Falls das in die Propandehydrierung rückgeführte Kreisgas Kohlenmonoxid enthält, kann dieses, bevor mit frischem Propan ergänzt wird, katalytisch zu CO₂ verbrannt werden. Die dabei freigesetzte Reaktionswärme kann zum Aufheizen auf die Dehydriertemperatur Anwendung finden.

Eine katalytische Nachverbrennung von im Restgas enthaltenem CO zu CO₂ kann auch dann empfehlenswert sein, wenn eine Abtrennung der Kohlenoxide aus dem Restgas vor dessen Rückführung als Kreisgas in die Propandehydrierung angestrebt wird, läßt sich doch CO₂ vergleichsweise einfach abtrennen (z.B. durch Wäsche mit einer basischen Flüssigkeit).

Natürlich kann auch so verfahren werden, daß man einen Teil des Restgases unverändert in die Propandehydrierung rückführt und nur aus dem verbliebenen Teil Propan und Propen im Gemisch abtrennt und ebenfalls in die Propandehydrierung und/oder in die Reaktionszone B rückführt. Im letzteren Fall vereint man den verbliebenen Teil des Restgases zweckmäßigerweise mit dem Produktgasgemisch A bzw. A'.

Im Rahmen einer fraktionierten Destillation des Restgases kann die Trennlinie z.B. so gelegt werden, daß im Auftriebsteil der Rektifikationskolonne im wesentlichen alle diejenigen Bestandteile abgetrennt werden und am Kopf der Kolonne abgezogen werden können, deren Siedepunkt tiefer als der Siedepunkt von Propen liegt. Diese Bestandteile werden in erster Linie die Kohlenoxide CO und CO₂ sowie nicht umgesetzter Sauerstoff und Ethylen sowie Methan und N₂ sein.

Als erfindungsgemäß wesentlich sei nochmals festgehalten, dass das im Normalbetrieb in die Reaktionszone A rückgeführte, Propan enthaltende, Restgas während einer Nichtbetriebsphase in der Reaktionszone A wenigstens teilweise (im Fall eines einzigen Reaktors A vorzugsweise vollständig) auf anderem Weg als über die Reaktionszone A in die Reaktionszone B rückgeführt wird und man gleichzeitig den Propenausfall aus einer anderen Quelle wenigstens teilweise und bevorzugt vollständig ergänzt.

Sowohl diese Ergänzung als auch die Rückführung erfolgen in diesem Fall vorzugsweise so, dass für den Fall, dass im Normalbetrieb nach der Reaktionszone A eine Abtrennvorrichtung integriert ist, auch diese Ergänzung und diese Rückführung während dieser Nichtbetriebsphase diese Abtrennvorrichtung durchlaufen müssen. Selbstredend kann sowohl diese Ergänzung als auch diese Rückführung auch hinter der Abtrennvorrichtung erfolgen.

Häufig wird das erfindungsgemäße Verfahren im Fall der Acrylsäureherstellung aus Propan so durchgeführt, daß im Produktgasgemisch B wenigstens 70 mol-%, vorzugsweise wenigstens 80 mol-% des in den verschiedenen Reaktionszonen insgesamt zugeführten molekularen Sauerstoff umgesetzt worden sind.

Vorzugsweise wird dabei in der zweiten Oxidationszone der Reaktionszone B bei einem molaren Acrolein : molekularem Sauerstoff : Wasserdampf : Propan : molekularem Stickstoff : sonstige Verdünnungsgase Verhältnis von 1 : 0,5 bis 1,5 : 0,1 bis 2 : 0,5 bis 6 : 1 bis 10 : 0 bis 5 gearbeitet.

Dabei ist es von Vorteil, wenn in der ersten Oxidationszone der Reaktionszone B Multimetalloxidkatalysatoren verwendet werden, die jenen der allgemeinen Formel I oder II oder III aus der DE-A 19 910 506 entsprechen und wenn in der zweiten Oxidationszone der Reaktionszone B Multimetalloxidkatalysatoren verwendet werden, die jenen der allgemeinen Formel I oder I' oder II aus der DE-A 19 910 508 entsprechen.

Als erfindungsgemäß geeignete Katalysatorgeometrien kommen dabei für die erste bzw. zweite Oxidationszone diejenigen in Betracht, die die DE-A 19 910 506 bzw. die DE-A 19 910 508 empfehlen.

Ferner können dabei die für die Reaktionszone B empfohlenen Rohrbündelreaktoren, was die Stromführung von Reaktionsgas und Temperiermedium (z.B. Salzbad) anbetrifft, sowohl im Gleichstrom als auch im Gegenstrom betrieben werden. Selbstredend können auch Querstromführungen überlagert werden. Besonders günstig ist eine mäanderförmige Führung des Temperiermediums um die Kontaktrohre herum, die über den Reaktor betrachtet wiederum im Gleichstrom oder im Gegenstrom zum Reaktionsgasgemisch erfolgen kann.

In der Regel werden für die Reaktionszone A Reaktoren mit passivierten Innenwandungen eingesetzt. Die Passivierung kann z.B. dadurch erfolgen, daß auf die Innenwandung vorab der Dehydrierung gesintertes Aluminiumoxid aufgebracht wird.
Sie kann aber auch in situ durch Zusatz geringer Mengen passivierender Hilfsstoffe (z.B. Sulfide) zum Reaktionsgasgemisch bewirkt werden.

Das erfindungsgemäße Verfahren ist dann bevorzugt, wenn ein Produktgemisch A' angewandt wird, das im wesentlichen nur wenigstens einen paraffinischen Kohlenwasserstoff A und wenigstens einen paraffinischen Kohlenwasserstoff A' enthält. Selbstverständlich kann zur Nebenkomponentenabtrennung beim erfindungsgemäßen Verfahren auch ein purge-Strom eingesetzt werden.

### Beispiele

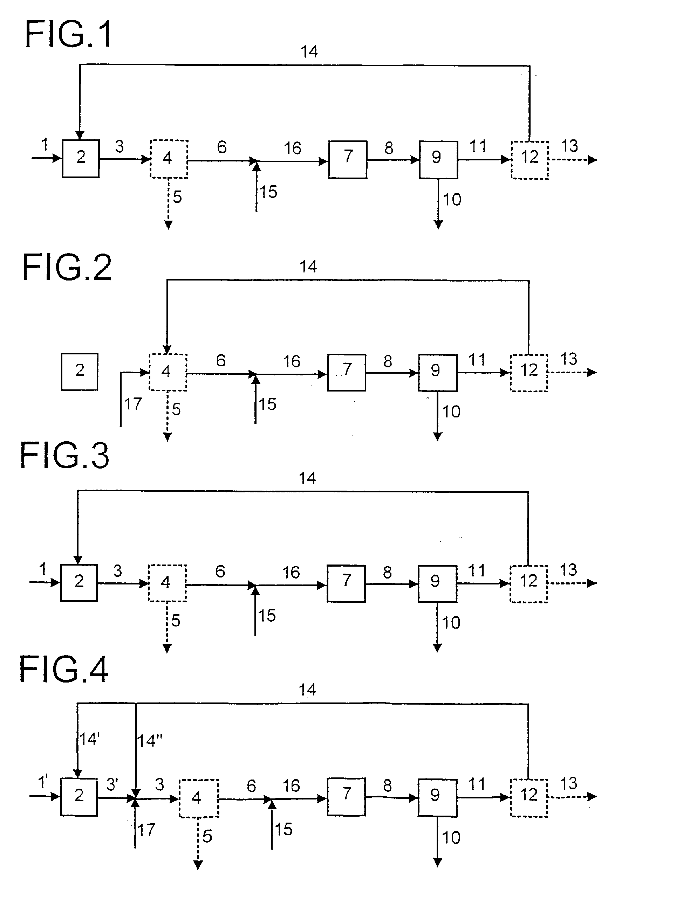

Die dieser Schrift beiliegende Figur 1 zeigt schematisch eine erfindungsgemäße Verfahrensweise im Normalbetrieb. Die Reaktionszone A soll nur einen Reaktor A umfassen.

Die dieser Schrift beiliegende Figur 2 zeigt eine zur Figur 1 gehörende erfindungsgemäße Nichtbetiebsphase. Dabei stehen die Ziffern für:
- 1 =: frischer paraffinischer Kohlenwasserstoff A und gegebenenfalls sonstige für die Dehydrierung und/oder Oxidehydrierung verwendete Ausgangsstoffe;
- 2 =: Reaktionszone A (für Oxidehydrierung und/oder Dehydrierung);
- 3 =: Produktgasgemisch A, enthaltend paraffinischen Kohlenwasserstoff A und olefinischen Kohlenwasserstoff A';
- 4 =: gegebenenfalls mitverwendete Abtrennzone zur Abtrennung von von paraffinischem Kohlenwasserstoff A und olefinischem Kohlenwasserstoff A' verschiedenen Nebenkomponenten;
- 5 =: in der Abtrennzone 4 gegebenenfalls abgetrennte Nebenkomponenten;
- 6 =: in Abhängigkeit einer Anwendung von 4 entweder Produktgemisch A oder Produktgemisch A';
- 16=: Beschickungsgemisch der Reaktionszone B;
- 15=: zusätzlich zum Produktgemisch A oder Produktgemisch A' für die Herstellung von 16 erforderliche Ausgangsstoffe (z.B. molekularer Sauerstoff);
- 7 =: Reaktionszone B;
- 8 =: Produktgemisch B;
- 9 =: Zielproduktabtrennung aus Produktgemisch B;
- 10=: Zielprodukt;
- 11=: paraffinischen Kohlenwasserstoff A enthaltendes Restgemisch;
- 12=: gegebenenfalls angewandte Abtrennvorrichtung für von paraffinischem Kohlenwasserstoff A verschiedene Nebenkomponenten;
- 13=: gegebenenfalls abgetrennte Nebenprodukte;
- 14=: paraffinischen Kohlenwasserstoff A enthaltender Rückführstrom;
- 17=: aus anderer Quelle als Reaktionszone A stammender olefinischer Kohlenwasserstoff A'.

Die dieser Schrift beiliegende Figur 3 zeigt schematisch eine erfindungsgemäße Verfahrensweise im Normalbetrieb. Die Reaktionszone A soll mehr als einen Reaktor A umfassen.

Die dieser Schrift beiliegende Figur 4 zeigt eine zur Figur 3 gehörende erfindungsgemäße Nichtbetriebsphase. Identische Ziffern haben die gleiche Bedeutung wie in den Figuren 1 und 2. Im übrigen stehen die nachfolgenden Ziffern für:
- 1'=: eine dem Nichtbetriebsanteil in der Reaktionszone A entsprechend geringere Menge an frischem paraffinischem Kohlenwasserstoff A und an gegebenenfalls sonstigen für die Dehydrierung und/oder Oxidehydrierung verwendeten Ausgangsstoffen;
- 3'=: reduzierte Menge an Produktgasgemisch A, enthaltend paraffinischen Kohlenwasserstoff A und olefinischen Kohlenwasserstoff A';
- 17=: aus anderer Quelle als Reaktionszone A stammender olefinischer Kohlenwasserstoff A', der die Minderproduktion in 2 kompensiert;
- 14'=: entsprechend dem Nichtbetriebsanteil in 2 verminderte Menge an paraffinischem Kohlenwasserstoff A enthaltendem Rückführstrom;
- 14"=: der Anteil des den paraffinischen Kohlenwasserstoff A enthaltenden Rückführstroms, der dem Nichtbetriebsanteil in 2 entspricht (14'+14" ≈14).

## Patentansprüche

1. Verfahren zur Herstellung von partiellen Oxidationsprodukten und/oder partiellen Ammoxidationsprodukten wenigstens eines olefinischen Kohlenwasserstoffs A', bei dem man
A) in einer ersten Reaktionszone A wenigstens einen paraffinischen Kohlenwasserstoff A einer partiellen Dehydrierung und/oder Oxidehydrierung unter Bildung eines Produktgemisches A, das eine nicht umgesetzte Menge des wenigstens einen paraffinischen Kohlenwasserstoff A und wenigstens einen durch die partielle Dehydrierung und/oder Oxidehydrierung gebildeten olefinischen Kohlenwasserstoff A' enthält, unterwirft,
B) aus dem Produktgemisch A von den darin enthaltenen, von dem wenigstens einen paraffinischen Kohlenwasserstoff A und dem wenigstens einen olefinischen Kohlenwasserstoff A' verschiedenen Bestandteilen gegebenenfalls in einer Abtrennzone eine Teil- oder die Gesamtmenge unter Erhalt eines Produktgemisches A' abtrennt und entweder das Produktgemisch A oder das Produktgemisch A' zur Beschikkung einer Oxidations- und/oder Ammoxidationszone B verwendet und in der Oxidations- und/oder Ammoxidationszone B wenigstens den wenigstens einen olefinischen Kohlenwasserstoff A' einer partiellen Oxidation und/oder Ammoxidation unter Bildung eines Produktgemisches B, das als Zielprodukt wenigstens ein partielles Oxidations- und/oder Ammoxidationsprodukt B des wenigsten einen olefinischen Kohlenwasserstoff A' enthält, unterwirft,
C) aus dem Produktgemisch B in einer Aufarbeitungszone C Zielprodukt abtrennt und von dem im Produktgemisch B enthaltenen, nicht umgesetzten wenigstens einen paraffinischen Kohlenwasserstoff A wenigstens eine Teilmenge als paraffinischen Rückführkohlenwasserstoff A in die Reaktionszone A zurückführt und
D) von Zeit zu Zeit in einer Nichtbetriebsphase wenigstens eine Teilmenge der Reaktionszone A nicht zum Zweck der partiellen Dehydrierung und/oder Oxidehydrierung des wenigstens einen paraffinischen Kohlenwasserstoff A betreibt, **dadurch gekennzeichnet, dass** man während der Nichtbetriebsphase der wenigstens einen Teilmenge der Reaktionszone A die Oxidations- und/oder Ammoxidationszone B weiter betreibt und dabei den mit der Nichtbetriebsphase verbundenen Produktionsausfall an dem wenigstens einen olefinischen Kohlenwasserstoff A' **dadurch** wenigstens teilweise kompensiert, dass man der Oxidations- und/oder Ammoxidationszone B wenigstens einen aus einer anderen Quelle als der Reaktionszone A stammenden olefinischen Kohlenwasserstoff A' und gegebenenfalls einen oder mehrere aus einer anderen Quelle als der Reaktionszone A stammenden paraffinischen Kohlenwasserstoff A zuführt und von dem dann im Produktgemisch B der Oxidations- und/oder Ammoxidationszone B befindlichen wenigstens einen paraffinischen Kohlenwasserstoff A wenigstens eine Teilmenge als paraffinischen Rückführkohlenwasserstoff A nicht über die Reaktionszone A in die Oxidations- und/oder Ammoxidationszone B rückführt.

2. Verfahren nach Anspruch 1, **dadurch gekennzeichnet, dass** die Reaktionszone A nur einen Reaktor A zur partiellen Dehydrierung und/oder Oxidehydrierung umfasst.

3. Verfahren nach Anspruch 2, **dadurch gekennzeichnet, dass** man den wenigstens einen paraffinischen Kohlenwasserstoff A in dem einen Reaktor A einer heterogen katalysierten partiellen Dehydrierung unterwirft.

4. Verfahren nach einem der Anprüche 1 bis 3**, dadurch gekennzeichnet, dass** der wenigsten eine paraffinische Kohlenwasserstoff A Propan und der wenigstens eine olefinische Kohlenwasserstoff A' Propylen ist.

5. Verfahren nach einem der Ansprüche 1 bis 4**, dadurch gekennzeichnet, dass** aus dem Produktgemisch A von den darin enthaltenen, von dem wenigstens einen paraffinischen Kohlenwasserstoff A und dem wenigstens einen olefinischen Kohlenwasserstoff A' verschiedenen, Bestandteilen in einer Abtrennzone wenigstens eine Teilmenge unter Erhalt eines Produktgemisches A' abgetrennt wird.

6. Verfahren nach einem der Ansprüche 1 bis 5**, dadurch gekennzeichnet, dass** aus dem Produktgemisch A von den darin enthaltenen, von dem wenigstens einen paraffinischen Kohlenwasserstoff A und dem wenigstens einen olefinischen Kohlenwasserstoff A' verschiedenen, Bestandteilen in einer Abtrennzone im wesentlichen die Gesamtmenge unter Erhalt eines Produktgemisches A' abgetrennt wird.

7. Verfahren nach Anspruch 5 oder 6, **dadurch gekennzeichnet, dass** man den aus einer anderen Quelle als der Reaktionszone A stammenden olefinischen Kohlenwasserstoff A' der Oxidations- und/oder Ammoxidationszone B so zuführt, dass er auch die Abtrennzone durchläuft.

8. Verfahren nach Anspruch 7, **dadurch gekennzeichnet, dass** man von dem im Produktgemisch B der Oxidations- und/oder Ammoxidationszone B befindlichen wenigstens einen paraffinischen Kohlenwasserstoff A die wenigstens eine Teilmenge so unter Ausschluß der Reaktionszone A in die Oxidations- und/oder Ammoxidationszone B rückführt, daß sie auch die Abtrennzone durchläuft.

9. Verfahren nach einem der Ansprüche 1 bis 8, **dadurch gekennzeichnet, dass** der aus einer anderen Quelle als der Reaktionszone A stammende olefinische Kohlenwasserstoff A' Propylen der Qualität chemical grade und/oder polymer grade ist.

10. Verfahren nach einem der Ansprüche 1 bis 9, **dadurch gekennzeichnet, dass** es ein Verfahren zur Herstellung von Acrolein und/oder Acrylsäure ist.

## Claims

1. A process for the preparation of partial oxidation products and/or partial ammoxidation products of at least one olefinic hydrocarbon A', in which
A) in a first reaction zone A, at least one paraffinic hydrocarbon A is subjected to a partial dehydrogenation and/or oxydehydrogenation with formation of a product mixture A which comprises an unconverted amount of the at least one paraffinic hydrocarbon A and at least one olefinic hydrocarbon A' formed by the partial dehydrogenation and/or oxydehydrogenation,
B) a portion or the total amount of the components present in the product mixture A and differing from the at least one paraffinic hydrocarbon A and the at least one olefinic hydrocarbon A' is separated from said product mixture A, if appropriate in a separation zone, to give a product mixture A', and either the product mixture A or the product mixture A' is used for feeding an oxidation and/or ammoxidation zone B and, in the oxidation and/or ammoxidation zone B, at least the at least one olefinic hydrocarbon A' is subjected to a partial oxidation and/or ammoxidation with formation of a product mixture B which comprises, as desired product, at least one partial oxidation and/or ammoxidation product B of the at least one olefinic hydrocarbon A',
C) the desired product is separated from the product mixture B in a working-up zone C and at least a portion of the unconverted at least one paraffinic hydrocarbon A present in the product mixture B is recycled as paraffinic recycle hydrocarbon A into the reaction zone A and
D) from time to time, in a nonoperating phase, at least a part of the reaction zone A is not operated for the purpose of the partial dehydrogenation and/or oxydehydrogenation of the at least one paraffinic hydrocarbon A, wherein, during the nonoperating phase of the at least one part of the reaction zone A, the oxidation and/or ammoxidation zone B is further operated and that production loss of the at least one olefinic hydrocarbon A' which is associated with the nonoperating phase is at least partly compensated by feeding at least one olefinic hydrocarbon A' originating from a source other than the reaction zone A and, if appropriate, one or more paraffinic hydrocarbons A originating from a source other than the reaction zone A to the oxidation and/or ammoxidation zone B and then recycling at least a portion of the at least one paraffinic hydrocarbon A present in the product mixture B of the oxidation and/or ammoxidation zone B, as paraffinic recycle hydrocarbon A, not via the reaction zone A, into the oxidation and/or ammoxidation zone B.

2. The process according to claim 1, wherein the reaction zone A comprises only one reactor A for the partial dehydrogenation and/or oxydehydrogenation.

3. The process according to claim 2, wherein the at least one paraffinic hydrocarbon A is subjected to a heterogeneously catalyzed partial dehydrogenation in the one reactor A.

4. The process according to any of claims 1 to 3, wherein the at least one paraffinic hydrocarbon A is propane and the at least one olefinic hydrocarbon A' is propylene.

5. The process according to any of claims 1 to 4, wherein at least a portion of the components present in the product mixture A and differing from the at least one paraffinic hydrocarbon A and the at least one olefinic hydrocarbon A' is separated from said product mixture A in a separation zone to give a product mixture A'.

6. The process according to any of claims 1 to 5, wherein substantially the total amount of the components present in the product mixture A and differing from the at least one paraffinic hydrocarbon A and the at least one olefinic hydrocarbon A' is separated from said product mixture A in a separation zone to give a product mixture A'.

7. The process according to claim 5 or 6, wherein the olefinic hydrocarbon A' originating from a source other than the reaction zone A is fed to the Oxidation and/or ammoxidation zone B in such a way that it also passes through the separation zone.

8. The process according to claim 7, wherein the at least one portion of the at least one paraffinic hydrocarbon A present in the product mixture B of the oxidation and/or ammoxidation zone B is recycled to the oxidation and/or ammoxidation zone B, with exclusion of the reaction zone A, in such a way that it also passes through the separation zone.

9. The process according to any of claims 1 to 8, wherein the olefinic hydrocarbon A' originating from a source other than the reaction zone A is chemical grade and/or polymer grade propylene.

10. The process according to any of claims 1 to 9, which is a process for the preparation of acrolein and/or acrylic acid.

## Revendications

1. Procédé de fabrication de produits partiels d'oxydation et/ou de produits partiels d'ammoxydation d'au moins un hydrocarbure oléfinique A', selon lequel
A) dans une première zone de réaction A, au moins un hydrocarbure paraffinique A est soumis à une déshydrogénation et/ou oxydéshydrogénation partielle avec formation d'un mélange de produits A qui contient une quantité non réagie du ou des hydrocarbures paraffiniques A et au moins un hydrocarbure oléfinique A' formé par la déshydrogénation et/ou oxydéshydrogénation partielle,
B) une partie ou la totalité des constituants différents du ou des hydrocarbures paraffiniques A et du ou des hydrocarbures oléfiniques A', présents dans le mélange de produits A, est séparée du mélange de produits A, éventuellement dans une zone de séparation, avec obtention d'un mélange de produits A' et le mélange de produits A ou le mélange de produits A' est utilisé pour l'alimentation d'une zone B d'oxydation et/ou d'ammoxydation et, dans la zone B d'oxydation et/ou d'ammoxydation, au moins le ou les hydrocarbures oléfiniques A' sont soumis à une oxydation et/ou ammoxydation partielle avec formation d'un mélange de produits B, qui contient en tant que produit cible au moins un produit d'oxydation et/ou d'ammoxydation partielle B du ou des hydrocarbures oléfiniques A',
C) le produit cible est séparé du mélange de produits B dans une zone de traitement C et au moins une partie du ou des hydrocarbures paraffiniques A non réagis présents dans le mélange de produits B est reconduite dans la zone de réaction A en tant qu'hydrocarbure paraffinique A recyclé et
D) de temps en temps, pendant une phase non opérationnelle, au moins une partie de la zone de réaction A n'est pas utilisée dans le but de la déshydrogénation et/ou oxydéshydrogénation partielle du ou des hydrocarbures paraffiniques A, **caractérisé en ce que** pendant la phase non opérationnelle d'au moins une partie de la zone de réaction A, la zone d'oxydation et/ou d'ammoxydation B est encore utilisée et la chute de production du ou des hydrocarbures oléfiniques A' associée à la phase non opérationnelle est au moins partiellement compensée en alimentant la zone d'oxydation et/ou d'ammoxydation B avec au moins un hydrocarbure oléfinique A' issu d'une autre source que la zone de réaction A et éventuellement avec un ou plusieurs hydrocarbures paraffiniques A issus d'une autre source que la zone de réaction A et au moins une partie du ou des hydrocarbures paraffiniques A se trouvant dans le mélange de produits B de la zone d'oxydation et/ou d'ammoxydation B est reconduite dans la zone d'oxydation et/ou d'ammoxydation B en tant qu'hydrocarbure paraffinique A recyclé, sans passer par la zone de réaction A,.

2. Procédé selon la revendication 1, **caractérisé en ce que** la zone de réaction A comprend uniquement un réacteur A pour la déshydrogénation et/ou oxydéshydrogénation partielle.

3. Procédé selon la revendication 2, **caractérisé en ce que** le ou les hydrocarbures paraffiniques A sont soumis à une déshydrogénation partielle sous catalyse hétérogène dans le réacteur A.

4. Procédé selon l'une quelconque des revendications 1 à 3, **caractérisé en ce que** le ou les hydrocarbures paraffiniques A sont le propane et le ou les hydrocarbures oléfiniques A' sont le propylène.

5. Procédé selon l'une quelconque des revendications 1 à 4, **caractérisé en ce qu'**au moins une partie des constituants différents du ou des hydrocarbures paraffiniques A et du ou des hydrocarbures oléfiniques A', présents dans le mélange de produits A, est séparée du mélange de produits A dans une zone de séparation, avec obtention d'un mélange de produits A'.

6. Procédé selon l'une quelconque des revendications 1 à 5, **caractérisé en ce qu'**essentiellement la totalité des constituants différents du ou des hydrocarbures paraffiniques A et du ou des hydrocarbures oléfiniques A', présents dans le mélange de produits A, est séparée du mélange de produits A dans une zone de séparation, avec obtention d'un mélange de produits A'.

7. Procédé selon la revendication 5 ou 6, **caractérisé en ce que** l'hydrocarbure oléfinique A' issu d'une autre source que la zone de réaction A est introduit dans la zone d'oxydation et/ou d'ammoxydation B de manière à ce qu'il traverse également la zone de séparation.

8. Procédé selon la revendication 7, **caractérisé en ce qu'**au moins une partie du ou des hydrocarbures paraffiniques A se trouvant dans le mélange de produits B de la zone d'oxydation et/ou d'ammoxydation B est reconduite dans la zone d'oxydation et/ou d'ammoxydation B lors de l'exclusion de la zone de réaction A de manière à ce qu'elle traverse également la zone de séparation.

9. Procédé selon l'une quelconque des revendications 1 à 8, **caractérisé en ce que** l'hydrocarbure oléfinique A' issu d'une autre source que la zone de réaction A est du propylène de grade chimique et/ou de grade polymère.

10. Procédé selon l'une quelconque des revendications 1 à 9, **caractérisé en ce** le procédé est un procédé de fabrication d'acroléine et/ou d'acide acrylique.
